(19) Europäisches Patentamt
European Patent Office
Office européen des brevets

(11) EP 3 419 977 B1

(12) EUROPEAN PATENT SPECIFICATION

(45) Date of publication and mention of the grant of the patent:
**03.01.2024 Bulletin 2024/01**

(21) Application number: **17711336.2**

(22) Date of filing: **23.02.2017**

(51) International Patent Classification (IPC):
***C07D 471/22*** *(2006.01)* ***A61K 31/439*** *(2006.01)*
***A61P 35/00*** *(2006.01)*

(52) Cooperative Patent Classification (CPC):
**C07D 471/22; A61K 31/437; A61K 45/06; A61P 35/00;** A61K 9/0014

(86) International application number:
**PCT/IB2017/051045**

(87) International publication number:
**WO 2017/145092 (31.08.2017 Gazette 2017/35)**

# (54) PLATINUM(II) RING-FUSED CHLORINS, METHODS AND USES THEREOF

PLATIN(II)-CHLORINE MIT KONDENSIERTEN RINGEN, VERFAHREN UND VERWENDUNGEN DAVON

CHLORINES FUSIONNÉES À UN CYCLE PLATINE (II), PROCÉDÉS ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.02.2016 PT 2016109182**

(43) Date of publication of application:
**02.01.2019 Bulletin 2019/01**

(73) Proprietor: **Universidade de Coimbra**
**3004-531 Coimbra (PT)**

(72) Inventors:
- **DOUGLAS BURROWS, Hugh**
  **3004-531 Coimbra (PT)**
- **VASCONCELOS DIAS DE PINHO E MELO, Teresa Margarida**
  **3004-531 Coimbra (PT)**
- **COIMBRA SERRA, Arménio**
  **3004-531 Coimbra (PT)**
- **MELO PEREIRA, Nelson António**
  **3004-531 Coimbra (PT)**
- **MARQUES MARTELO, Liliana**
  **3004-531 Coimbra (PT)**
- **PIÑERO GÓMEZ, Marta**
  **3004-531 Coimbra (PT)**
- **SEIXAS DE MELO, João Sérgio**
  **3004-531 Coimbra (PT)**
- **FERREIRA PITA BATISTA PINA, João Manuel**
  **3004-531 Coimbra (PT)**
- **RABAÇA ROQUE BOTELHO, Maria Filomena**
  **3004-531 Coimbra (PT)**
- **COELHO ABRANTES, Ana Margarida**
  **3004-531 Coimbra (PT)**
- **LARANJO, Mafalda**
  **3004-531 Coimbra (PT)**

(74) Representative: **Patentree**
**Edificio Net**
**Rua de Salazares, 842**
**4149-002 Porto (PT)**

(56) References cited:
- **PEREIRA NELSON A M ET AL: "Novel 4,5,6,7-tetrahydropyrazolo[1,5-a]pyridine fused chlorins as very active photodynamic agents for melanoma cells", EUROPEAN JOURNAL OF MEDICINAL CHEMISTRY, vol. 103, 2015, pages 374-380, XP029295251, ISSN: 0223-5234, DOI: 10.1016/J.EJMECH.2015.08.059**
- **SHENGLIN LUO ET AL: "A review of NIR dyes in cancer targeting and imaging", BIOMATERIALS., vol. 32, no. 29, 1 October 2011 (2011-10-01), pages 7127-7138, XP055309265, GB ISSN: 0142-9612, DOI: 10.1016/j.biomaterials.2011.06.024 cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **NELSON A. M. PEREIRA ET AL: "Platinum(II) Ring-Fused Chlorins as Near-Infrared Emitting Oxygen Sensors and Photodynamic Agents", ACS MEDICINAL CHEMISTRY LETTERS, vol. 8, no. 3, 22 February 2017 (2017-02-22), pages 310-315, XP055366701, United States ISSN: 1948-5875, DOI: 10.1021/acsmedchemlett.6b00476**

## Description

### Technical Field

The present disclosure relates to derivatives of platinum (II) ring-fused chlorins with the general formula I more particularly derivatives, which are novel and stable near infrared luminescent compounds for imaging, sensing, optoelectronics applications and use in medicine or as a medicament.

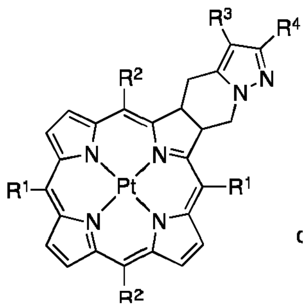

(formula I)

This disclosure describes a series of novel, stable near infrared luminescent compounds based on platinum (II) ring-fused chlorins with the general formula I, in particular platinum (II) derivatives of 4,5,6,7-tetrahydropyrazolo[1,5-$\alpha$]pyridine-fused chlorins. These compounds show good light emission, in particular simultaneous fluorescence and phosphorescence emission in solution at room temperature, in the biologically important 700-850 nm near-infrared (NIR) spectral region. This, coupled with excellent thermal and photochemical stability, makes them excellent materials for use in biological imaging, oxygen sensing and as emitting materials in optoelectronic applications, such as organic light emitting diodes (OLEDs) or bulk heterojunction photovoltaic cells. In addition, their chemical properties allow them to be chemically attached to specific biologically relevant molecules for targeted applications in imaging and theranostics.

### Background

Molecular imaging is becoming increasingly important in clinical diagnosis and biomedical research for the study of processes at the cellular and subcellular levels, with much effort being placed on targeting and reporting disease and metabolism in vivo.[1] Imaging techniques are typically considered in terms of the region of the electromagnetic spectrum involved, with the most important methods corresponding to radiation in the radiofrequency (magnetic resonance imaging), visible/near infrared (optical imaging) and high energy X-rays and $\gamma$-rays (X-ray computed tomography, positron emission tomography, and single-photon emission computed tomography) regions.[2] Optical imaging using luminescent materials is of particular relevance for biological applications since it is relatively low cost, non-invasive, requires low cost equipment and has reasonable sensitivity and good temporal resolution.[1] Near infrared (NIR) emitters are particularly important for these applications since their light output is in a region where organisms are highly transparent (the optical or therapeutic window). Although the optical window in biological materials typically extends from 600 to 1450 nm [2] the major interest focuses on light emitters in the therapeutically relevant 700-900 nm region [3]. At these wavelengths there is little light absorption by cell constituents, such as proteins, hemoglobin, melanin and water, and light scattering is reduced compared with the visible region. In addition, there are other spectral advantages including increased signal-to-noise ratio resulting from decreased interference from other light sources. Particularly important applications include NIR luminescence imaging of cells for diagnostic studies of cancer and other conditions. [4-7] The most important classes of NIR emitters are inorganic fluorophores, such as quantum dots, or upconverting fluorophores containing lanthanide ions, and organic dyes, such as cyanines, squaraines, phthalocyanines, porphyrins, boron dipyrromethanes (BODIPYs), perylene dyes and carbon nanotubes.[2,6] These can be conjugated to appropriate ligands or antibodies for targeting specific sites of biological interest.[6] In addition, fluorescent proteins are being developed which emit in the near infrared region. [2] However, until now the only long wavelength emission compound approved by the US Food and Drug agency (FDA) for direct usage in medical diagnostics is the cyanine dye indocyanine green.[2] There is, therefore, the urgent need to develop new materials with this properties.

These facts are presented in order to illustrate the technical problem addressed by the present disclosure.

### General Description

There is a major advantage if the same luminescent materials which are used in imaging can also be used as

fluorescent probes and sensors for both qualitative and quantitative analysis of chemical species. One important target is molecular oxygen, whose concentration can provide information on cell state. Quenching of excited states of compounds by molecular oxygen can lead to reactive oxygen species, such as singlet oxygen or superoxide radical anion, which can kill malignant cells. Combining this capacity with the diagnostic capability means that these systems have potential as theranostic agents.

The optimal requirements for an NIR probe or imaging agent for biological systems are high molar absorption coefficient, good luminescence quantum yield, large Stokes shift between absorption and emission maxima, good chemical and photochemical stability, low toxicity, and the possibility of dissolving in aqueous media. In addition, it is a strong advantage if it can be readily conjugated through covalent bonding with specific molecules, such as aminoacids, proteins, nucleic acids, for targeting specific biological sites.

Near infrared emitters also have potential in materials areas, such as light emitting diodes and photovoltaic cells. The chemical, spectral and photochemical requirements for these applications are similar to those for molecular imaging.

The incorporation of high atomic number metals, such as platinum, can enhance the triplet state properties of porphyrins, phthalocyanines, chlorins and bacteriochlorins, and in many cases lead to long wavelength, room temperature phosphorescence. However, photostability is a huge problem that limits the manipulation and use of these compounds. A platinum complex of a tetrabenzoporphyrin showed NIR phosphorescence and good stability.

In general chlorins and bacteriochlorins are porphyrin derivatives with good spectral and photophysical characteristics for use as near infrared luminescent compounds. In particular, the platinum derivatives of these classes of compounds are very important. However, chlorins and bacteriochlorins prepared by the classical route of porphyrin reduction are very unstable under ambient conditions, and tend to be rapidly oxidized back to the porphyrin precursor or to degrade by oxygen to other kind of macrocycles. Chlorins and bacteriochlorins with particular structures that avoid the oxidation back to porphyrin or the degradative process are required in order to produce efficient near infrared luminescent compounds with high enough stability to be used in imaging and theranostics or other applications, which require prolonged contact with air.

In an embodiment, new near infrared luminescent oxygen sensors based relates to platinum(II) derivatives of ring-fused chlorins with the general formula I is reported. Their high thermal and photochemical stability, attractive photophysical features and oxygen dependent room temperature phosphorescence, make them excellent compounds to be used as probes for molecular oxygen, biological imaging and photodynamic therapy. This implies that the described compounds are true, stable Pt-chlorin-type theranostic agents.

The present disclosure relates to a new class of near infrared light emitter (NIR) based on platinum(II) complexes of 4,5,6,7-tetrahydropyrazolo[1,5-*a*]pyridine-fused chlorins with structure I.

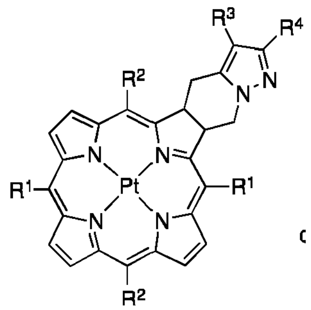

(formula I)

An aspect of the present disclosure relates to compound of formula I wherein

- $R^1$ and $R^2$ are aryl groups,
- $R^3$ and $R^4$ are selected from the following list: ester group, or a hydroxymethyl group, preferably wherein the ester group is a methyl ester group,

for use in the treatment, therapy or diagnostic of a neoplasia or an infectious diseases.

The term "aryl group" as used herein is any carbon-based aromatic group including, but not limited to, benzene, naphthalene, etc. The term "aromatic" also includes "heteroaryl group," which is defined as an aromatic group that has at least one heteroatom incorporated within the ring of the aromatic group. Examples of heteroatoms include, but are not limited to, nitrogen, oxygen, sulfur, and phosphorus. The aryl group can be substituted or unsubstituted. The aryl group can be substituted with one or more groups including, but not limited to, alkyl, alkynyl, alkenyl, aryl, halide, nitro,

amino, ester, ketone, aldehyde, hydroxy, carboxylic acid, or alkoxy.

"Aryl" means mono- or bicyclic aromatic rings containing only carbon atoms or a fused analog thereof. A "fused analog" means mono- or bicyclic aromatic rings containing only carbon atoms fused to a monocyclic cycloalkyl or monocyclic heterocyclic group in which the point of attachment is on the aromatic portion. Examples of aryl and fused analogs thereof include phenyl, naphthyl, indanyl, indenyl, tetrahydronaphthyl, 2,3-dihydrobenzofuranyl, dihydrobenzopyranyl, 1 ,4-benzodioxanyl, and the like.

"Heteroaryl" means a mono- or bicyclic aromatic ring containing at least one heteroatom selected from N, O and S, with each ring containing 5 to 6 atoms, or a fused analog thereof. A "fused analog" means a mono- or bicyclic aromatic ring containing at least one heteroatom selected from N, O and S, with each ring containing 5 to 6 atoms, fused to a monocyclic cycloalkyl or monocyclic heterocyclic group in which the point of attachment is on the aromatic portion. Examples of heteroaryl include pyrrolyl, isoxazolyl, isothiazolyl, pyrazolyl, pyridyl, oxazolyl, oxadiazolyl, thiadiazolyl, thiazolyl, imidazolyl, triazolyl, tetrazolyl, furanyl, triazinyl, thienyl, pyrimidyl, pyridazinyl, pyrazinyl, benzoxazolyl, benzothiazolyl, benzimidazolyl, benzofuranyl, benzothiophenyl, furo(2,3-b)pyridyl, quinolyl, indolyl, isoquinolyl, and the like.

Where elements are presented as lists, e.g., in Markush group format, it is to be understood that each subgroup of the elements is also disclosed, and any element(s) can be removed from the group. It is also noted that the term "comprising" is intended to be open and permits the inclusion of additional elements or steps. It should be understood that, in general, where the invention, or aspects of the invention, is/are referred to as comprising particular elements, features, steps, etc., certain embodiments of the invention or aspects of the invention consist, or consist essentially of, such elements, features, steps, etc. For purposes of simplicity those embodiments have not been specifically set forth in haec verba herein. Thus, for each embodiment of the invention that comprises one or more elements, features, steps, etc., the invention also provides embodiments that consist or consist essentially of those elements, features, steps, etc.

In an embodiment, the aryl group is a phenyl, naphthyl, or combinations thereof.

In an embodiment, the phenyl group is an unsubstituted phenyl or a substituted phenyl.

In an embodiment, the naphthyl group is an unsubstituted naphthyl or a substituted naphthyl.

In an embodiment, the substituted phenyl group or the substituted naphthyl group or the substituted benzoyl group is substituted with one or more of the groups consisting of an alkyl, alkoxy, alkylcarboxy, alkylcarbonyl, alkyl ester, cyano, halogen or haloalkyl.

In an embodiment, the substituted phenyl is group 4-chlorophenyl (4-$ClC_6H_4$), 4-methylphenyl (4-$MeC_6H_4$), 4-methoxyl (4-$MeOC_6H_4$), 4-fluorophenyl (4-$FC_6H_4$), 3-methylphenyl (3-$MeC_6H_4$) or 2,6-dichlorophenyl (2,6-$Cl_2C_6H_3$).

In an embodiment, the $R^3$ and $R^4$ is $CO_2Me$, or $CH_2OH$.

In an embodiment, the substituted phenyl group is 4-chlorophenyl (4-$ClC_6H_4$), 4-methylphenyl (4-$MeC_6H_4$), 4-methoxyl (4-$MeOC_6H_4$), 4-fluorophenyl (4-$FC_6H_4$), 3-methylphenyl (3-$MeC_6H_4$) or 2,6-dichlorophenyl (2,6-$Cl_2C_6H_3$) group and the $R^3$ and $R^4$ is $CO_2Me$.

In an embodiment, the substituted phenyl group is 4-chlorophenyl (4-ClC6$H_4$), 4-methylphenyl (4-$MeC_6H_4$), 4-methoxyl (4-$MeOC_6H_4$), 4-fluorophenyl (4-$FC_6H_4$), 3-methylphenyl (3-$MeC_6H_4$) or 2,6-dichlorophenyl (2,6-$Cl_2C_6H_3$) group and the $R^3$ and $R^4$ is $CH_2OH$.

In an embodiment, the ester group is a methyl ester group.

In an embodiment, the compound is platinum (II) dimethyl 4,5,6,7-tetrahydropyrazolo[1,5-$\alpha$]pyridine-2,3-dicarboxylate-fused chlorin.

In an embodiment, the compound is platinum (II) 2,3-dihydroxymethyl-4,5,6,7-tetrahydropyrazolo[1,5-$\alpha$]pyridine-fused chlorin.

In an embodiment, the new compounds of the present disclosure are for use in medicine or veterinary, in particular for use in the treatment, diagnostic, therapy of a disease characterized by benign or malignant cellular hyperproliferation or by areas of neovascularisation or a cancer.

In an embodiment, the new compounds of the present disclosure may be use in treatment, diagnostic, therapy of infectious diseases that are caused by microorganisms including viruses, bacteria, rickettsia, mycoplasma, protozoa, fungi, or parasites; in particular HIV virus or flu virus.

In an embodiment, the new compounds of the present disclosure may be used in treatment, diagnostic, or detection of hyperproliferative tissue, in particular hyperproliferative tissue associated to cancer, carcinoma, myeloma, psoriasis, macular degeneration, and/or precancerous conditions. In particular, the new compounds may be use in photodynamic therapy or cytoluminescent therapy.

In an embodiment, the new compounds of the present disclosure may be administrated by oral, parenteral, intramuscular, intranasal, sublingual or intratracheal route. In particular the new compounds may be administrated 1 to 100 hours before photodynamic therapy or cytoluminescent therapy, preferably 3 to 60 hours before therapy (for ex.: irradiation), more preferably 10-40 hours before therapy (for ex.: irradiation).

Photodynamic therapy is a treatment that uses special drugs, called photosensitizing agents, along with light to kill cancer cells. The drugs only work after they have been activated or "turned on" by certain kinds of light. Photodynamic therapy may also be called photo-chemotherapy.

Another aspect of the present disclosure relates to pharmaceutical composition comprising at least one of the new compounds of the present disclosure.

In an embodiment, the pharmaceutical composition may comprises a pharmaceutically acceptable carrier; in particular wherein the pharmaceutically acceptable carrier is transient permeabilizer of the skin.

In an embodiment, the pharmaceutical composition may further comprise a surface penetration enhancer; in particular wherein the surface penetration enhancer is selected from a list consisting of: dialkylsulphoxides, N-methylformamide, dimethylformamide, dimethylacetamide, glycols, pyrrolidone derivatives of 1 -substituted azacycloalkan-2-ones, or mixtures thereof, among others.

In an embodiment, the pharmaceutical composition may further comprises an anti-viral, an analgesic, an anti-inflammatory agent, a chemotherapy agent, a radiotherapy agent, an antibiotic or a diuretic, among others.

In an embodiment, the pharmaceutical composition may further comprise a filler, a binder, a disintegrant or a lubricant, among others.

In an embodiment, the pharmaceutical composition may be used in intradermal and transdermal therapies.

In an embodiment, the pharmaceutical composition may be used in the treatment, therapy or diagnostic of hyperproliferative disorders including benign tumour, cancer, carcinoma, myeloma, macular degeneration, and precancerous conditions, skin disorder selected from actinic keratoses, squamous cell carcinoma, Bowen’s disease, psoriasis, acne vulgaris and rosacea.

In an embodiment, the pharmaceutical composition may be used in the treatment, therapy or diagnostic of cancer, malignant tumour, early cancer, basal cell carcinoma, cervical dysplasia, soft tissue sarcoma, germ cell tumour, retinoblastoma, age-related macular degeneration; Hodgkin’s lymphoma, cancer of the blood, prostate cancer, cervix cancer, uterus cancer, vaginal cancer, breast cancer, naso-pharynx cancer, trachea cancer, larynx cancer, bronchi cancer, bronchioles cancer, lung cancer, hollow organs cancer, esophagus cancer, stomach cancer, bile duct cancer, intestine cancer, colon cancer, colorectum cancer, rectum cancer, bladder cancer, ureter cancer, kidney cancer, liver cancer, gall bladder cancer, spleen cancer, brain cancer, lymphatic system cancer, bone cancer or skin cancer.

In an embodiment, the pharmaceutical composition may be used in the treatment, therapy or diagnostic or detection a dermatological condition, acne, or psoriasis.

In an embodiment, the pharmaceutical composition may be used in the treatment, therapy or diagnostic or detection of a fungal, viral, chlamydial, bacterial, nanobacterial or parasitic infectious disease.

In an embodiment, the pharmaceutical composition may be used in the treatment, therapy of HIV, infection with SARS coronavirus, Asian flu virus, herpes simplex or herpes zoster; hepatitis; viral hepatitis.

Another aspect of the present invention, is related to a kit comprising a new compound and/or a pharmaceutical composition of the present disclosure. In particular, a kit further comprising instructions for photodynamic therapy or cytoluminescent therapy, and/or instructions for administration of the composition to a subject.

The compounds of the present disclosure may be administered enterically, orally, parenterally, sublingually, by inhalation (e. g. as mists or sprays), rectally, or topically in dosage unit formulations containing conventional nontoxic pharmaceutically or physiologically acceptable carriers, adjuvants, and vehicles as desired. For example, suitable modes of administration include oral, subcutaneous, transdermal, transmucosal, iontophoretic, intravenous, intraarterial, intramuscular, intraperitoneal, intranasal (e. g. via nasal mucosa), subdural, rectal, gastrointestinal, and the like, and directly to a specific or affected organ or tissue. For delivery to the central nervous system, spinal and epidural administration, or administration to cerebral ventricles, can be used. Topical administration may also involve the use of transdermal administration such as transdermal patches or iontophoresis devices. The term parenteral as used herein includes subcutaneous injections, intravenous, intramuscular, intrasternal injection, or infusion techniques. The compounds are mixed with pharmaceutically acceptable carriers, adjuvants, and vehicles appropriate for the desired route of administration. The compounds described for use in medicine can be administered in solid form, in liquid form, in aerosol form, or in the form of tablets, pills, powder mixtures, capsules, granules, injectables, creams, solutions, suppositories, enemas, colonic irrigations, emulsions, dispersions, food premixes, and in other suitable forms. The compounds can also be administered in liposome formulations. The compounds can also be administered as prodrugs, where the prodrug undergoes transformation in the treated subject to a form which is therapeutically effective. Additional methods of administration are known in the art.

Injectable preparations, for example, sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent, for example, as a solution in propylene glycol. Among the acceptable vehicles and solvents that may be employed are water, Ringer’s solution, and isotonic sodium chloride solution. In addition, sterile, fixed oils are conventionally employed as a solvent or suspending medium. For this purpose any bland fixed oil may be employed including synthetic mono- or diglycerides. In addition, fatty acids such as oleic acid find use in the preparation of injectables.

Solid dosage forms for oral administration may include capsules, tablets, pills, powders, and granules. In such solid dosage forms, the active compound may be admixed with at least one inert diluent such as sucrose, lactose, or

starch. Such dosage forms may also comprise additional substances other than inert diluents, e.g., lubricating agents such as magnesium stearate. In the case of capsules, tablets, and pills, the dosage forms may also comprise buffering agents. Tablets and pills can additionally be prepared with enteric coatings.

Liquid dosage forms for oral administration may include pharmaceutically acceptable emulsions, solutions, suspensions, syrups, and elixirs containing inert diluents commonly used in the art, such as water. Such compositions may also comprise adjuvants, such as wetting agents, emulsifying and suspending agents, cyclodextrins, and sweetening, flavoring, and perfuming agents.

Examples of dosages which can be used are an effective amount of the compounds of the invention within the dosage range of about 0.1 μg/kg to about 300 mg/kg, or within about 1.0 μg /kg to about 40 mg/kg body weight, or within about 1.0 μg/kg to about 20 mg/kg body weight, or within about 1.0 μg /kg to about 10 mg/kg body weight, or within about 10.0 μg /kg to about 10 mg/kg body weight, or within about 100 μg/kg to about 10 mg/kg body weight, or within about 1.0 mg/kg to about 10 mg/kg body weight, or within about 10 mg/kg to about 100 mg/kg body weight, or within about 50 mg/kg to about 150 mg/kg body weight, or within about 100 mg/kg to about 200 mg/kg body weight, or within about 150 mg/kg to about 250 mg/kg body weight, or within about 200 mg/kg to about 300 mg/kg body weight, or within about 250 mg/kg to about 300 mg/kg body weight. Other dosages which can be used are about 0.01 mg/kg body weight, about 0.1 mg/kg body weight, about 1 mg/kg body weight, about 10 mg/kg body weight, about 20 mg/kg body weight, about 30 mg/kg body weight, about 40 mg/kg body weight, about 50 mg/kg body weight, about 75 mg/kg body weight, about 100 mg/kg body weight, about 125 mg/kg body weight, about 150 mg/kg body weight, about 175 mg/kg body weight, about 200 mg/kg body weight, about 225 mg/kg body weight, about 250 mg/kg body weight, about 275 mg/kg body weight, or about 300 mg/kg body weight. Compounds of the present invention may be administered in a single daily dose, or the total daily dosage may be administered in divided dosage of two, three or four times daily.

The present disclosure also relates to a new synthetic process for obtaining the compounds now disclosed involving an unprecedented step consisting of [8π+2π] cycloaddition of diazafulvenium methides to platinium complexes of porphyrins.

In an embodiment, the compounds of the present disclosure have good light emission properties in the 700-750 nm spectral region.

In an embodiment, the compounds of the present disclosure are characterized by the intensity of luminescence showing unprecedented sensitivity to molecular oxygen.

In an embodiment, the compounds of the present disclosure are near infrared light emitters with excellent photostability.

In an embodiment, the compounds of the present disclosure have exceptional spectral properties for use as near infrared light emitters for biological and biomedical probes and imaging.

In an embodiment the compounds of the present disclosure have exceptional spectral properties for use as emitting materials in organic light emitting diodes (OLEDs).

In an embodiment, the compounds of the present disclosure provide the potential for in vivo sensing and imaging through steady state luminescence and for whole cell imaging through time resolved fluorescence microscopy (FLIM).

In an embodiment, the compounds of the present disclosure are characterized by the capacity of quenching of excited states by molecular oxygen to generate reactive oxygen species, making these materials relevant for photodynamic therapy.

Another aspect of the present disclosure relates to the use of the new compounds of the present disclosure as a molecular oxygen sensor or as biological/biomedical probe, or as a film, or as an OLED.

## Brief Description of the Drawings

The following figures provide preferred embodiments for illustrating the description and should not be seen as limiting the scope of disclosure.

Figure 1: Absorption spectra of chlorin **7b** and **8** (solid and dotted lines) and TPP as reference (dashed line) in toluene solution.

Figure 2: Fluorescence spectra of chlorin **7b** and TPP in a toluene solution.

Figure 3: Phosphorescence spectrum and decay of chlorin **7** in degassed toluene solution.

Figure 4: Emission spectra for the chlorin **7b** in toluene (dashed line) and in a deaerated toluene solution (solid line).

Figure 5: Phosphorescence emission of the chlorin 8 in ethanol solution at various times in the presence (A) and in the absence of the oxygen (B).

Figure 6: Singlet oxygen phosphorescence of the chlorin **8** in ethanol.

Figure 7: Normalized absorption spectra of Pt(II) chlorins **7b** and **8,** presented with TPP and Pt(II)-TPP (considered as references compounds) in toluene solution at T = 293K.

Figure 8: Room temperature luminescence spectra ($\lambda_{exc}$ = 400 nm) for chlorins **7b** and **8** in oxygen saturated toluene solutions prepared by bubbling oxygen for 3 hours.

Figure 9: Room temperature luminescence spectra ($\lambda_{exc}$ = 410 nm) together with the normalized absorption spectra for Pt(II) chlorin **7b** and **8** in aqueous micellar system (Tween80/DMSO/water (2/2/96, v/v/v)), collected in the presence (air saturated solution) and absence of $O_2$ (after deoxygenation by bubbling $N_2$ for 20 min). Inset: Magnified view of the fluorescence emission bands present in the 590-700 nm range.

Figure 10: Plot of 1°(755 nm/600 nm)/1(755 nm/600 nm) as function of $O_2$ concentration - from 0 to 21 % (gas phase) - for chlorin **6** in aqueous micellar system (Tween80/DMSO/water (2/2/96, v/v/v)).

Figure 11: Metabolic activity of A375 human melanoma cells submitted to photodynamic treatment using the photosensitizer chlorins **7b** and 8. The values represent the average and standard deviation for each concentration.

Figure 12: (A) Room temperature luminescence spectra ($\lambda_{exc}$ = 410 nm) for Pt(II)-chlorin **7** in toluene solution in the presence (air saturated solution) and absence of $O_2$ (after deoxygenation by bubbling $N_2$ for 20 min). Also shown as inset is a magnified view of the fluorescence emission bands present in the 575-700 nm range; (B) Room temperature luminescence spectra for Pt(II)-chlorin **8** collected with $\lambda_{exc}$ = 413 nm in nitrogen saturated toluene solution, together with the normalized phosphorescence band obtained in the ns-TA setup for comparison purposes.

Figure 13: Phosphorescence decays of the Pt(II)-chlorin **8** in DMSO:Tween80:H2O (2:2:96, v/v/v) solution in the presence (A) and in the absence of oxygen (B). The decay data analysis was performed with a single exponential fit, giving a phosphorescence lifetime of 11.3 and 30.9 $\mu$s respectively.

Figure 14: (A, B) Room temperature time-resolved phosphorescence spectrum for chlorins **7** and **8** in degassed toluene solutions obtained in the ns-TA setup; together with the (C, D) phosphorescence decays for chlorin **6** in toluene and tris(2,2'-bipyridyl)ruthenium (II), $Ru(bpy)_3$, in water, collected at $\lambda_{em}$= 755 nm and 620 nm, respectively, in air equilibrated and degassed solutions (after bubbling with $N_2$ for 30 min); Also shown as inset in (C) are the room temperature luminescence spectra for $Ru(bpy)_3$ in water collect in aerated and $O_2$ saturated solution (by bubbling $O_2$ for 3 hours). The time resolved phosphorescence spectra for chlorins 7 and **8** were also obtained in a nanosecond transient absorption setup (ns-TA, operating in emission mode) equipped with an Hamamatsu R5509-42 photomultiplier cooled to 193 K in a liquid nitrogen chamber (Products for Research model PC176TSCE-005).

Figure 15: Isotermal thermogravimetric analysis of chlorin **7b** (green) and chlorin **8** (red). The thermal stability of chlorins **7b** and **8** was evaluated by isotermal thermogravimetric analysis of the samples at 60 °C during 3 hours. Negligible mass loss (less than 1%) was observed for the two studied compounds.

Figure 16: Room temperature singlet oxygen phosphorescence emission spectra for chlorin **7b** together with the reference compound phenazine in air saturated ethanol solutions. Singlet oxygen formation on photolysis of aerated solution of chlorin **8** was demonstrated by its near infrared phosphorescence around 1270 nm. Phenazine was used as the standard for the determination of singlet oxygen formation quantum yields ($\phi_\Delta$).

**Detailed Description**

The present disclosure relates to derivatives of platinum (II) ring-fused chlorins with the general formula I, more particularly derivatives, which are novel and stable near infrared luminescent compounds for imaging, sensing, optoelectronics applications and use in medicine or as a medicament.

More in particular a new class of platinum (II) derivatives of 4,5,6,7-tetrahydropyrazolo[1,5-*a*]pyridine-fused chlorins which possess appropriate spectral and photophysical properties, coupled with high thermal and photochemical stability, making them excellent materials for use in molecular imaging, oxygen sensing and optoelectronics. These will be exemplified by the Pt complex of the dimethyl ester of the 4,5,6,7-tetrahydropyrazolo[1,5-*a*]pyridine-fused chlorin and the 2,3-dihydroxymethyl-4,5,6,7-tetrahydropyrazolo[1,5-*a*]pyridine-fused chlorin as typical members of the class of materials described in this disclosure.

The preparation of the compounds of the present disclosure is given below.

This disclosure relates to a new and unprecedented [8π+2π] cycloaddition of diazafulvenium methides with porphyrins and chlorins [11,12]. It was observed that porphyrins reacted with diazafulvenium methide **2,** generated *in situ* from 2,2-dioxo-1*H*,3*H*-pyrazolo[1,5-c]thiazoles **1** by thermal extrusion of sulfur dioxide, affording 4,5,6,7-tetrahydropyrazolo[1,5-*a*]pyridine-fused chlorin derivatives **4** in a selective fashion. Interestingly, the cycloaddition of **2** was more efficient with *meso*-tetraphenylporphyrin **(3b)** giving chlorin **4b** in 31% yield. The formation of bis adducts (bacteriochlorins) was never detected. The best reaction conditions to obtain chlorins **4** were achieved by carrying out the reaction with excess of porphyrin (2 equiv) under microwave irradiation for 20 min at 250 °C **(Scheme 1).**

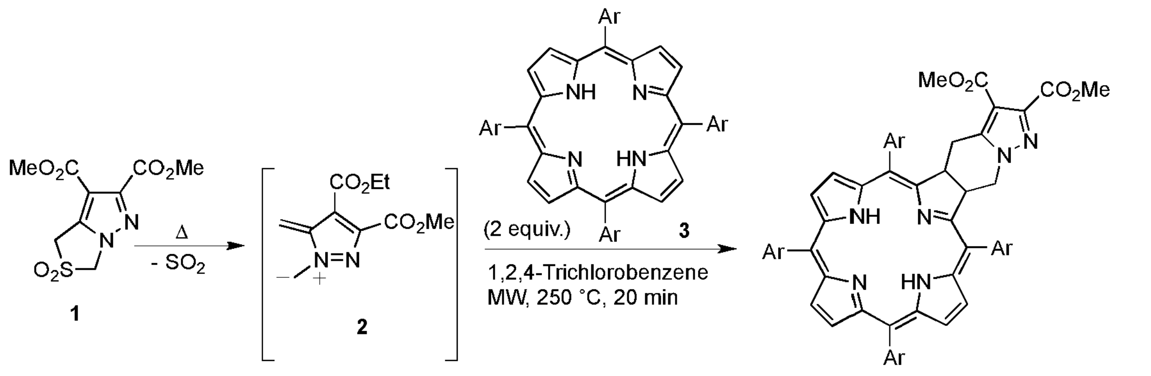


| | Yield | Porphyrin Recovered |
|---|---|---|
| **4a** Ar = 4-$ClC_6H_4$ | 30% | [65%] |
| **4b** Ar = Ph | 31% | [58%] |
| **4c** Ar = 4-$MeC_6H_4$ | 26% | [51%] |
| **4d** Ar = 4-$MeOC_6H_4$ | 20% | [48%] |
| **4e** Ar = 4-$FC_6H_4$ | 10% | [34%] |
| **4f** Ar = 3-$MeOC_6H_4$ | 13% | [51%] |
| **4g** Ar = 2,6-$Cl_2C_6H_4$ | 13% | [41%] |

**Scheme 1.**

The [8π+2π] cycloaddition of diazafulvenium methides for the preparation of bacteriochlorins was also explored [12] **(Scheme 2).** The microwave induced reaction of chlorin **4a** with diazafulvenium methide **2** was carried out under various reaction conditions. In this case, conventional heating proved to be more efficient than the microwave methodology. Carrying out the reaction in refluxing 1,2,4-trichlorobenzene for 4 h, the target bacteriochlorin **5a** was isolated as single product in 36% yield (40% calculated based on the chlorin consumed). The recovery of chlorin reagent at the end of this reaction is an indication of the considerable stability of this macrocycle. The [8π+2π] cycloaddition reaction was carried out with other chlorins, namely meso-tetraphenyl-, *meso-tetrakis*(4-methylphenyl)- and *meso-tetrakis*(4-methoxyphenyl)-substituted derivatives **4b-4d.** The target bacteriochlorins **5b-5d** were obtained in yields ranging from 16 to 29%. No evidence for the formation of isobacteriochlorin was observed. Thus, the cycloaddition of *meso*-tetraarylchlorins with diazafulvenium methides to give 4,5,6,7-tetrahydropyrazolo[1,5-*a*]pyridine-fused bacteriochlorins is site-, regio- and stereoselective. Detailed absorption and fluorescence spectral and quantum yield measurements have been made on all new chlorins and bacterichlorins. Although these compounds show potential for various biomedical applications, a further step is needed to develop these materials for application as NIR emitters.

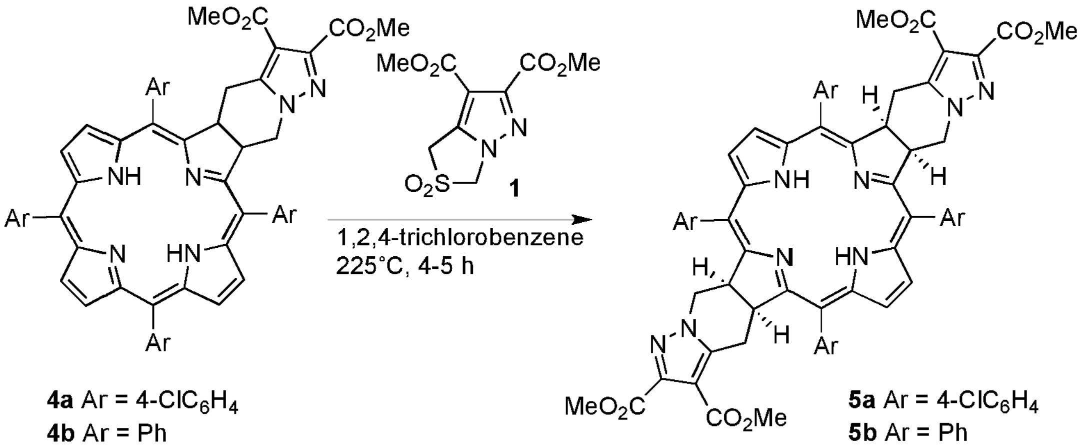


**4a** Ar = 4-$ClC_6H_4$
**4b** Ar = Ph
**4c** Ar = 4-$MeC_6H_4$
**4d** Ar = 4-$MeOC_6H_4$

**5a** Ar = 4-$ClC_6H_4$ 36%
**5b** Ar = Ph 29%
**5c** Ar = 4-$MeC_6H_4$ 27%
**5d** Ar = 4-$MeOC_6H_4$ 16%

**Scheme 2.**

The great stability of the above 4,5,6,7-tetrahydropyrazolo[1,5-a]pyridine-fused chlorins has led us to prepare Pt(II) complexes of these derivatives. Initially, insertion of Pt(II) into chlorin **4b** was attempted. However, using the classical way to introduce metal in the porphyrin core by reacting chlorin **4b** with Pt(II) chloride (4-5 equiv ) either using conventional heating (refluxing benzonitrile, 3 h) or under microwave irradiation (250 ºC, 4-10 min) only produced trace amounts of the target metallated chlorin. It was clear that to get the required platinum derivatives some novel strategy must be envisaged. Hence, these results led us to devise a different synthetic strategy, which is described for the first time in this patent. First, the platinum complex of *meso*-tetraphenyl porphyrin **(6)** was prepared following a known general procedure, replacing, however, $Pt(acac)_2$ as the Pt(II) source with $PtCl_2$[16]. Thus, *meso*-tetraphenyl porphyrin **(3b)** reacted with $PtCl_2$ in benzonitrile under microwave irradiation at 250 °C for 20 min giving the Pt-complex **6** in 90% yield. The metallated porphyrin **6** (2 equiv.) reacted with 2,2-dioxo-1*H*,3*H*-pyrazolo[1,5-c]thiazoles **1** under microwave irradiation at 225 °C for 20 min giving the novel Pt complex of 4,5,6,7-tetrahydropyrazolo[1,5-*a*]pyridine-fused chlorin **7b** in 20% yield (50% of porphyrin recovered). Using the same strategy chlorin **7a** was also prepared from the corresponding Pt(II) complex of 5,10,15,20-tetrakis(4-chlorophenyl)porphyrin **(3a)** in 12% yield.

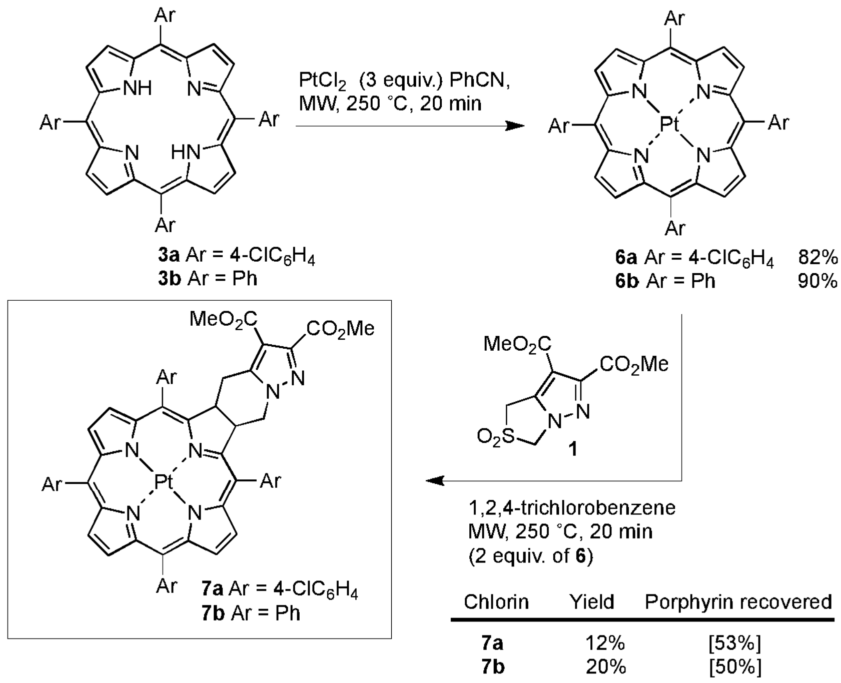


| Chlorin | Yield | Porphyrin recovered |
|---|---|---|
| **7a** | 12% | [53%] |
| **7b** | 20% | [50%] |

**Scheme 3.** Preparation of the Pt (II) chlorin **7.**

This strategy allows the preparation of the Pt complex of the chlorin and also proves the generality of the novel [8π+2π] cycloaddition reaction, which is also possible with the platinum complex.

In an embodiment and in order to improve the hydrophilicity of the complex for application in biological systems, Pt (II) chlorin **7** was reduced to the corresponding dihydroxymethyl derivative **8.** Reduction of chlorin **7** with an excess of $LiAlH_4$, at 50 °C, for 4 hours gave the corresponding di-hydroxyl complex **8** in 59% yield (Scheme 4). In addition to its effect in increasing hydrophilicity, the presence of the hydroxymethyl substituents will allow further functionalization to make conjugates with specific biologically relevant molecules for targeted applications in imaging and theranostics.

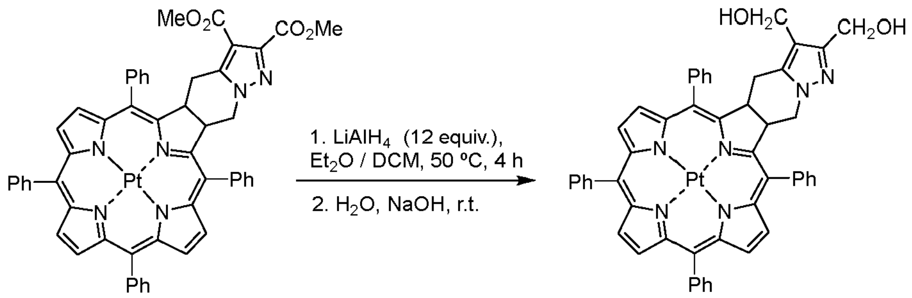


**7**

**8** 59%

**Scheme 4.** Preparation of the dihydroxymethyl substituted Pt (II) chlorin **8.**

A regioselective reduction of the ester function in **7b** to the mono-hydroxymethyl derivative **9** was achieved (scheme 5). Treatment of **7b** with six molar excess of lithium borohydride and methanol in refluxing tetrahydrofuran, afforded **9** in 55% yield. Dihydroxymethyl derivative **8** was also obtained as minor product in 19% yield.

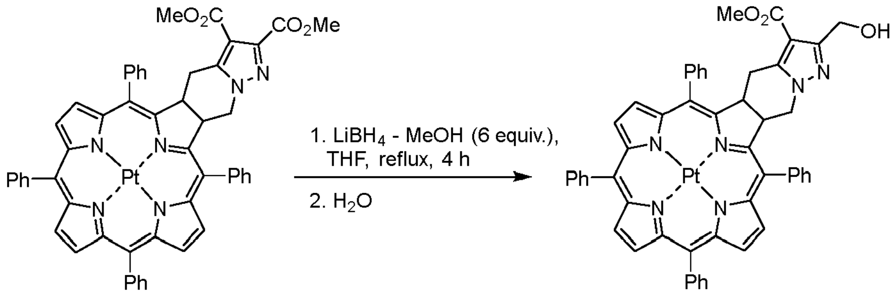


**7b**

**9** 55%

**Scheme 5.** Preparation of the mono-hydroxylmethyl substituted Pt(II) chlorin **9**.

In order to increase the hydrophilic character of the chlorin derivatives, chlorins bearing two carboxylic acid groups were prepared. In presence of KOH, the hydrolysis of diester chlorin **7b** took place to give the corresponding dicarboxylic acid-derivative **10** in 61% yield.

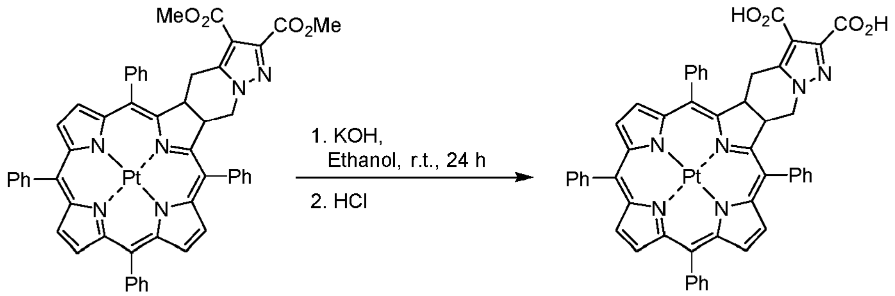


**7b**

**10** 61%

**Scheme 6.** Preparation of the dicarboxylic acid Pt(II) chlorin **1.**

The luminescence and absorption spectra of Pt complex of chlorin **7b** and **8** were analysed. The absorption spectrum of the Pt complex of chlorin **7b** and **8** (shown in toluene solution) has a first maximum at 590 nm, and a series of absorption bands at shorter wavelengths. The long wavelength absorption is much stronger than that of corresponding porphyrins, such as 5,10,15,20-tetratolylporphyrin (TTP) or their Pt(II) complexes.

In an embodiment, the chlorin **7b** shows a weak fluorescence when compared with the reference porphyrin TPP, with bands at 650 and 720 nm.

Unexpectedly, degassed solutions of chlorin **7b** show a very strong phosphorescence, with a maximum at 755 nm. This is an excellent region for NIR imaging and probing.

The absorption spectra of the Pt complexes **7b** and **8** (in toluene solution) display wavelength maxima at ~590 nm, together with additional absorption bands at shorter wavelengths (Figure 7 and Table 1). Note, for the compounds investigated the absorbance of the low energy band is much greater than those found for TPP or its Pt(II) complex (see Figure 7).

Figure 8 presents the emission spectra for chlorins **7b** and **8.** The fluorescence emission is structured, with a maxima at 598 nm (Table 1). However, one of the most interesting feature of these compounds is the additional occurrence of room temperature phosphorescence, with maxima at 755 nm (see Figure 8). The assignment of this long wavelength band to phosphorescence was based on its similarity with the RT phosphorescence emission spectra collected with 5 μs delay after flash and the time resolved phosphorescence spectra obtained in a laser flash photolysis setup (see Figures 8 and 12-13). For the Pt(II) derivatives (chlorins **7b** and **8**) at 293 K, the RT phosphorescence spectra display an unstructured long wavelength emission band centered at ~755 nm (Figures 12 and 14). This provides the possibilities for using these compounds for NIR imaging. There is a strong overlap between the phosphorescence and the fluorescence emission bands. However, the phosphorescence is highly sensitive to oxygen, and increases when the concentration of $O_2$ in solution is reduced. This allows determination of the fluorescence quantum yields for chlorins **7b** and **8** using oxygen saturated solutions, where the phosphorescence is effectively quenched, see Figure 8, while the fluoresecence is little affected. The fluorescence quantum yields ($\phi_F$) for chlorin **7b** and **8** were obtained using TPP ($\phi_F$= 0.11) in toluene as standard, showing a lower $\phi_F$ = 0.0001 value, see Table 1. This is particular relevant when compared to the free base chlorins of **7b** and **8** where the fluorescence quantum yields are more than 3-orders of magnitude higher. In addition, a

blue-shift of the fluorescence was also observed upon going from the free base chlorins to the corresponding Pt(II)-chlorin derivatives (see Table 1).

In an embodiment, degassed solutions of the Pt(II) chlorins in toluene show a much stronger phosphorescence at 756 nm compared with aerated ones, while the fluorescence emission intensity at 600 nm remains unaltered, as can be seen in Fig. 12. The phosphorescence quantum yields ($\phi_{Ph}$) for chlorins **7b** and **8** were obtained by using tris(2,2'-bipyridyl)ruthenium (II) in water ($\phi_F$= 0.042)[25] as standard. For further details please see the experimental Section in SI. The values of the phosphorescence quantum yields (Table 1) depend on the dissolved oxygen concentration in the solution, varying from 0.0002 in oxygen saturated toluene (3 hours of bubbling with oxygen); through 0.0028 with an air equilibrated solution ($[O_2]$= $1.8\times10^{-3}$ M), finally to 0.088 with a nitrogen saturated solution. The strong quenching of the phosphorescence band of chlorins **7b** and **8** by molecular oxygen, and the increase in its intensity by over two orders of magnitude on going from oxygen saturated to degassed solutions (see Table 1, Figure 12) opens the possibility of sensing oxygen in chemical and biological media by studying the ratio of the intensity of phosphorescence to fluorescence bands (at ~600 nm). One particularly important application of such ratiometric sensing is in cancer diagnosis, since it could, in principle, enable the determination of intracellular oxygen concentration, thereby allowing distinction between normoxic and hypoxic cells. This also opens the way for *in vivo* oxygen tumour imaging by phosphorescence quenching.

In an embodiment, to mimic biological conditions, a micellar system of the surfactant polysorbate 80 (Tween 80) in aqueous DMSO solution (Tween80/DMSO/water (2/2/96, v/v/v)) was used. In this medium similar spectroscopic properties (absorption and emission wavelength maxima) were observed as those in toluene solution spectra (Figure 9).

In an embodiment, the potential for oxygen sensing of chlorin **8** was analyzed quantitatively using phosphorescence intensities at different oxygen concentrations and the Stern-Volmer equation (eqn. 1).

$$\frac{I^{\circ}}{I} = 1 + K_{SV}pO_2 \quad \text{(eqn. 1)}$$

where $K_{SV}$ is the Stern-Volmer constant, $pO_2$ is the oxygen partial pressure, $I^0$ = (luminescence intensity at 755 nm / luminescence intensity at 600 nm) and $I$ = (luminescence intensity at 755 nm / luminescence intensity at 600 nm) at several $pO_2$ from 0 to 21%. A good linear fit was observed up to 21 % oxygen, with a $K_{SV}$ value of 0.18 (Figure 10). The fluorescence intensity monitored at 600 nm was practically unchanged, showing that chlorin **8** can be used as a ratiometric phosphorescence probe for measuring oxygen pressures and, consequently, solution concentrations.

In an embodiment, the phosphorescence lifetime of chlorin **8** was also determined in the presence and absence of oxygen in DMSO:Tween80:$H_2O$ (2:2:96) (Table 1), and changed from 11.3 $\mu$s (aerated solution with $O_2$) to 30.9 $\mu$s (degassed solution), see Figure 13A and 13B in SI. The observed decrease in phosphorescence lifetime makes this class of compounds good candidates for applications in phosphorescence lifetime imaging microscopy, in addition to their use as steady-state ratiometric $O_2$ sensor probes.

In an embodiment, the photostability of chlorin **8** was determined by monitoring the phosphorescence emission of a deaerated solution in water at 756 nm. The phosphorescence intensity at 756 nm remained unchanged over ~4.5 h irradiation. Finally, the thermal stability of chlorins **7b** and **8** was evaluated, using isothermal thermogravimetric analysis of the samples at 60 ºC during 3 h. Negligible mass loss (less than 1 %) was observed for the two compounds studied ( Fig. 15).

In an embodiment, these results demonstrate that chlorins **7b** and **8** have high thermal stability and photostability. Quenching of the phosphorescence may be expected to produce singlet oxygen ($^1\Delta_g$). Its formation was confirmed by direct measurement of the characteristic singlet oxygen phosphorescence emission centered at 1270 nm following irradiation of aerated solutions of chlorin **8.** The singlet oxygen quantum yield ($\phi_\Delta$) was obtained by comparing the sensitized phosphorescence emission spectra from singlet oxygen (Fig. 16), obtained with optically matched solutions of the samples and that of the reference phenazine.

In an embodiment, a value of $\phi_\Delta$=0.58 was obtained for chlorin **8.** This strongly suggests that the platinum(II) ring-fused chlorins can also be potentially interesting photosensitizers for photodynamic therapy.

In an embodiment, the photocytotoxicity of chlorins **7b** and **8** against human melanocytic melanoma cells (A375) was evaluated. It was observed that the di(hydroxymethyl)-substituted chlorin **8** shows high photodynamic activity with an $IC_{50}$ of 144 nM (confidence interval at 95 %: [121.8;171.0]) (Figure 5), comparable to the $IC_{50}$ value of 156 nM obtained for Photofrin@ under the same conditions. Additionally, this chlorin **8** was significantly more active than the diester-substituted chlorin **7b** ($IC_{50}$ > 5 $\mu$M) as was previously also observed for the corresponding metal-free chlorins. Experiments with A375 cells in the dark confirmed that the cytotoxicity is light-dependent. In the case of chlorin **8,** some cytotoxicity was observed in the absence of light, but with an $IC_{50}$ value of 22.29 $\mu$M. Thus, the potential of platinum (II) 4,5,6,7-tetrahydropyrazolo[1,5-*a*]pyridine-fused chlorins as photosensitizers for PDT of melanocytic melanoma cells has been demonstrated.

All spectral data are summarized in the Table below.

Table 1. Absorption and photophysical data (absorption, fluorescence and phosphorescence maxima, fluorescence $\Phi_F$ and phosphorescence quantum yields, $\Phi_{Ph}$ and phosphorescence lifetimes, $\tau_{Ph}$) for the Pt(II) chlorins and the corresponding free base chlorins in solution at room temperature.

| | [a]Absorption $\lambda_{max}$ (nm) | | | | | [a]Fluorescence $\lambda_{max}$ (nm) | | [a]Phosphorescence $\lambda_{max}$ (nm) | $\phi_F$ *,a | $\phi_P$ *,a | Phosporescence lifetime (μs) |
|---|---|---|---|---|---|---|---|---|---|---|---|
| | $Q_x$ (O-0) | $Q_x$ (1-0) | $Q_y$ (0-0) | $Q_y$ (1-0) | B (0-0) | Q (0-0) | Q (0-1) | T (0-0) | | | |
| Chlorin **7b** | - | 590 | 554 | 484 | 400 | 598 | 655 | 756 | 0.0001[e] | 0.0002[c]<br>0.0028[c]<br>0.088[d] | 0.4[a,c]<br>26.1[a,d] |
| Chlorin **8** | - | 591 | 555 | 486 | 400 | 598 | 644 | 756 | 0.0001[c] | 0.0002[e]<br>0.0019[c]<br>0.068[d] | 0.5[a,c]<br>25.7[a,d]<br>30.9[b,d]<br>11.3[b,c] |
| Free base chlorin of **7b** | 649 | 595 | 545 | 518 | 416 | 654 | 698, 721 | - | 0.27 | - | - |
| Free base chlorin of **6** | 651 | 601 | 543 | 514 | 409 | 654 | 696, 718 | - | 0.23 | - | - |

*Fluorescence quantum yields ($\lambda_{exc}$ = 413 nm) were determined by using TPP in toluene ($\Phi_F$ = 0.11)[24] and tris(2,2'-bipyridyl)ruthenium (II) in water ($\Phi_F$ = 0.042)[25] as a standard; [a]in toluene, [b]in aqueous micellar system (Tween80/DMSO/water (2/2/96, v/v/v)), [c]presence of $O_2$, [d]deaerated solution; [e] $O_2$ saturated solution; values presented in parenthesis were obtained in the ns-TA setup.

In an embodiment, the effect of oxygen was analysed. Oxygen concentrations are very different in normal and cancerous cells. The ratiometric luminescence method allows the determination of intracellular oxygen concentration for molecular diagnostics. In addition, differences in phosphorescent intensity as a consequence of oxygen quenching allow distinction between normal and malignant cells for biomedical imaging.

The intensity of the phosphorescence of chlorins **7** and **8** are strongly quenched by the presence of oxygen. In contrast, the fluorescence is relatively unaffected. This provides the possibilities of sensing oxygen in chemical and biological media. The fact that the fluorescence is little affected allows the possibility of ratiometric sensing, where the fluorescence band is used as a reference for calibrating oxygen induced decreases in the intensity of the phosphorescence band. Quantitative analysis of oxygen concentration is made by studying the ratio of the intensity of phosphorescence to fluorescence peaks. This can provide a quantitative measure of oxygen concentration inside cells. Finally, the phosphorescence lifetime is also reduced in the presence of oxygen, which makes this class of compounds suitable for applications in fluorescence lifetime imaging microscopy.

The phosphorescence lifetime of the chlorins **7b** and **8** were also determined **(Figure 5).** In **figure 5,** it can be observed that the phosphorescence lifetime is reduced in the presence of oxygen, and that a phosphorescence lifetime with oxygen of 865 ns **(Figure 5A)** was obtained and in a dearated ethanol solution of 9.40 μs **(Figure 5B).** The phosphorescence lifetime of chlorin **8** was also determined in the presence and absence of oxygen in DMSO:Tween80:$H_2O$ (2:2:96) (Table 1) and changed from 11.3 μs (aerated solution with $O_2$) to 30.9 μs (degassed solution).

Singlet oxygen formation on photolysis of aerated solutions of clorins **7** and **8** is demonstrated by near infrared phosphorescence **(Figure 6).** Phenazine was used as the standard for the determination of singlet oxygen formation quantum yields ($\Phi_\Delta$) [19]. For chlorin **8** a value of 0.58 was. This indicates the potential of these compounds also for applications in photodynamic therapy for various therapeutic applications. In addition, it supports the claim that the described compounds are likely to be useful for theranostics.

In an embodiment, the photostability of chlorin **8** was determined monitoring the phosphorescence emission of a deaerated solution in water at 756 nm. It was observed that the phosphorescence intensity at 756 nm remains unchanged during 4.5 h.

**Synthesis**

In an embodiment, the platinum (II) dimethyl 4,5,6,7-tetrahydropyrazolo[1,5-α]pyridine-2,3-dicarboxylate-fused chlorin (7) was synthetized as follows.

In an embodiment, a solution of 2,2-dioxo-1*H*,3*H*-pyrazolo[1,5-c][1,3]thiazole-6,7-dicarboxylate (**5,** 0.07 mmol) and the respective Pt(II) porphyrin **6** (0.14 mmol, 2 equiv ) in 1,2,4-trichlorobenzene (2 mL) was flushed with argon and irradiated in a microwave reactor (CEM Focused Synthesis System, Discover S-Class) with the temperature set to 250 °C for 20 min. After cooling to room temperature, some drops of triethylamine were added, and the mixture was purified by flash chromatography ($CH_2Cl_2$/ethyl acetate, 95:5) to afford the corresponding unreacted Pt(II) porphyrin **6** and Pt(II) chlorin **7.** Chlorin **7a** was obtained in 12% yield (47% of porphyrin **6a** recovered) and chlorin **7b** in 20% yield (50% of porphyrin **6b** recovered).

In an embodiment, the characterization of the platinum (II) dimethyl 4,5,6,7-tetrahydropyrazolo[1,5-*α*]pyridine-2,3-dicarboxylate-fused chlorins **7** was as follows: **7a** $^1$H NMR (400 MHz, $CDCl_3$): $\delta$ = 8.38 (s, 2H, *β*-H pyrrolic), 8.35 (2d, superimposed doublets, *J* = 5.0 Hz, 2H, *β*-H pyrrolic), 8.13 (d, *J* = 5.1 Hz, 1H, *β*-H pyrrolic), 8.10 (d, *J* = 5.1 Hz, 1H, β-H pyrrolic), 8.01-7.95 (m, 3H, Ar), 7.92-7.85 (m, 4H, Ar), 7.78-7.72 (m, 4H, Ar), 7.69-7.60 (m, 6H, Ar), 5.70-5.63 (m, 1H, reduced *β*-H pyrrolic), 5.38-5.31 (m, 1H, reduced *β*-H pyrrolic), 4.38 (dd, *J* = 13.6, 7.4 Hz, 1H, $CH_2$ fused ring), 3.89 (s, 3H, $CO_2Me$), 3.88 (dd, *J* = 13.6, 9.8 Hz, 1H, CH2 fused ring), 3.79 (s, 3H, $CO_2Me$), 3.54 (dd, *J* = 15.9, 6.7 Hz, 1H, $CH_2$ fused ring), 2.56 (dd, *J* = 15.9, 10.1 Hz, 1H, $CH_2$ fused ring) ppm. **7b** $^1$H NMR (400 MHz, $CDCl_3$): $\delta$ = 8.41 (s, 2H, *β*-H pyrrolic), 8.38-8.36 (m, 2H, *β*-H pyrrolic), 8.16-8.13 (m, 2H, *β*-H pyrrolic), 8.11-7.94 (m, 7H, Ar), 7.82-7.76 (m, 3H, Ar), 7.71-7.60 (m, 10H, Ar), 5.78-5.71 (m, 1H, reduced *β*-H pyrrolic), 5.39-5.32 (m, 1H, reduced *β*-H pyrrolic), 4.33 (dd, *J* = 13.5, 7.7 Hz, 1H, $CH_2$ fused ring), 3.93 (dd, *J* = 13.5, 9.8 Hz, 1H, $CH_2$ fused ring), 3.88 (s, 3H, $CO_2Me$), 3.74 (s, 3H, $CO_2Me$), 3.58 (dd, *J* = 15.8, 6.4 Hz, 1H, $CH_2$ fused ring), 2.64 (dd, *J* = 15.8, 10.4 Hz, 1H, $CH_2$ fused ring) ppm. Further, C NMR (100 MHz, $CDCl_3$): $\delta$ = 162.6, 162.2, 150.7, 148.4, 146.4, 146.2, 143.3, 143.0, 140.8, 140.6, 140.3, 138.4, 138.2, 135.9, 135.7, 134.5, 134.1, 133.3, 133.2, 132.3, 132.3, 131.6, 131.5, 128.9, 128.7, 128.6, 128.3, 128.1, 128.0, 127.7, 127.6, 127.5, 127.0, 126.9, 126.6, 126.1, 126.0, 113.5, 113.3, 110.7, 52.5, 51.6, 49.2, 45.6, 43.4, 26.2 ppm; ESI-HRMS found 1017.2585, calcd. 1017.2601 ($C_{53}H_{38}N_6O_4$Pt $M^+$).

**In** an embodiment, the platinum (II) 2,3-dihydroxymethyl-4,5,6,7-tetrahydropyrazolo[1,5-*α*]pyridine-fused chlorin **(8)** was synthetized as follows.

In an embodiment, a solution of Pt(II) chlorin **7b** (36 mg, 0.035 mmol) in dry dichloromethane (3 mL) was added dropwise to a stirred suspension of $LiAlH_4$ (16 mg, 0.421 mmol, 12 equiv ) in dry diethyl ether (3 mL), at 0 °C. After addition is complete, the mixture was heated to 50 °C and was left stirring at this temperature for 4 hours. The reaction mixture was cooled to 0 °C (ice bath) and quenched over a 30-min period with ethyl acetate (30 mL), water (10 mL) and

aqueous 10% sodium hydroxide (0.2 mL). Then the reaction mixture was warmed to room temperature and left stirring 16 hours. The two phases were separated in a separating funnel. The aqueous phase was extracted with ethyl acetate (30 ml). Combined organic extracts were dried ($Na_2SO_4$), reduced in volume and the product was purified by flash chromatography (ethyl acetate) to afford Pt (II) chlorin **8** (20 mg, 59%).

In an embodiment, the characterization of the platinum (II) 2,3-dihydroxymethyl-4,5,6,7-tetrahydropyrazolo[1,5-$\alpha$]pyridine-fused chlorin **(8)** was as follows: $^1$H NMR (400 MHz, $CDCl_3$): $\delta$ = 8.40 (s, 2H, $\beta$-H pyrrolic), 8.38-8.35 (m, 2H, $\beta$-H pyrrolic), 8.17-8.16 (m, 2H, $\beta$-H pyrrolic), 8.10-8.08 (m, 1H, Ar), 8.06-7.93 (m, 6H, Ar), 7.82-7.80 (m, 1H, Ar), 7.76-7.58 (m, 12H, Ar), 5.72-5.65 (m, 1H, reduced $\beta$-H pyrrolic), 5.35-5.29 (m, 1H, reduced $\beta$-H pyrrolic), 4.52 (s, 2H, $CH_2OH$), 4.22 (d, *J* = 12.6 Hz, 1H, $CH_2OH$), 4.15 (d, *J* = 12.6 Hz, 1H, $CH_2OH$), 4.10 (dd, *J* = 13.3, 7.8 Hz, 1H, $CH_2$ fused ring), 3.89 (dd, *J* = 13.3, 9.5 Hz, 1H, $CH_2$ fused ring), 2.91 (dd, *J* = 15.2, 6.3 Hz, 1H, $CH_2$ fused ring), 2.47 (dd, *J* = 15.2, 10.2 Hz, 1H, $CH_2$ fused ring) ppm. Further, C NMR (100 MHz, $CDCl_3$): $\delta$ = 151.4, 149.5, 149.3, 146.2, 146.1, 140.8, 140.5, 138.2, 138.1, 137.8, 135.8, 135.6, 134.7, 134.3, 133.2, 132.2, 132.1, 131.6, 131.5, 128.7, 128.6, 128.5, 128.1, 127.9, 127.6, 127.4, 127.3, 127.0, 126.6, 126.4, 126.0, 115.8, 113.5, 57.9, 54.2, 53.4, 48.5, 46.1, 44.3, 25.0 ppm; ESI-HRMS found 961.2691, calcd. 961.2702 ($C_{51}H_{38}N_6O_2Pt$ $M^+$).

In an embodiment, the platinum (II) 2-hydroxymethyl-3-methoxycarbonyl-4,5,6,7-tetrahydropyrazolo[1,5-$\alpha$]pyridine-fused chlorin **(9)** was synthetized as follows.

In an embodiment, to a solution of Pt(II) chlorin **7b** (11 mg, 0.011 mmol) in dry tetrahydrofuran (1 mL) was added $LiBH_4$ (1 mg, 0.060 mmol, 12 equiv) and methanol (3 $\mu$L, 0.060 mmol). The mixture was refluxed for 4 hours, then cooled to room temperature and quenched (ice bath cooling) with aqueous HCl (5%). The mixture was extracted with ethyl acetate (20 mL), the organic extract was dried (anhydrous $Na_2SO_4$) and the solvent was evaporated under reduced pressure. The product was purified by flash chromatography to afford Pt(II) chlorin **9** (6 mg, 55% [dichloromethane/ethyl acetate (8/2) as eluent] followed by Pt(II) chlorin **8** (2 mg, 19%) [ethyl acetate as eluent].

In an embodiment, the characterization of the platinum (II) 2-hydroxymethyl-3-methoxycarbonyl-4,5,6,7-tetrahydropyrazolo[1,5-$\alpha$]pyridine-fused chlorin **(9)** was as follows: $^1$H NMR (400 MHz, $CDCl_3$): $\delta$ = 8.41 (s, 2H, $\beta$-H pyrrolic), 8.38 (2d, superimposed doublets, *J* = 5.0 Hz, 2H, $\beta$-H pyrrolic), 8.18 (d, *J* = 5.0 Hz, 1H, $\beta$-H pyrrolic), 8.15 (d, *J* = 5.0 Hz, 1H, $\beta$-H pyrrolic), 8.12-7.95 (m, 7H, Ar), 7.87-7.85 (m, 1H, Ar), 7.82-7.76 (m, 2H, Ar), 7.72-7.65 (m, 10H, Ar), 5.72-5.65 (m, 1H, reduced $\beta$-H pyrrolic), 5.45-5.38 (m, 1H, reduced $\beta$-H pyrrolic), 4.69-4.59 (m, 2H, $CH_2OH$), 4.21 (dd, *J* = 13.5, 7.6 Hz, 1H, $CH_2$ fused ring), 3.83 (dd, *J* = 13.6, 9.8 Hz, 1H, $CH_2$ fused ring), 3.76 (s, 3H, $CO_2Me$), 3.61 (dd, *J* = 15.9, 6.9 Hz, 1H, $CH_2$ fused ring), 2.60 (dd, *J* = 15.9, 10.0 Hz, 1H, $CH_2$ fused ring) ppm.

In an embodiment, the platinum (II) 4,5,6,7-tetrahydropyrazolo[1,5-$\alpha$]pyridine-2,3-dicarboxylic acid-fused chlorin **(10)** was synthetized as follows.

In an embodiment, to a solution of Pt(II) chlorin **7b** (25 mg, 0.025 mmol) in ethanol (5 mL) was added a saturated KOH solution (0.5 mL). The mixture was left stirring at room temperature for 24 hours. The potassium carboxylate of the chlorin precipitated from reaction mixture and was filtered off. Then the precipitate was added to aqueous HCl (5%) and the dicarboxylic acid chlorin **10** was collected by filtration, washed with water and dried under vacuum. Pt (II) chlorin **10** was obtained in 61% (15 mg) yield.

In an embodiment, the characterization of the platinum (II) 4,5,6,7-tetrahydropyrazolo[1,5-$\alpha$]pyridine-2,3-dicarboxylic acid-fused chlorin **(10)** was as follows: $^1$H NMR (400 MHz, $CDCl_3$): $\delta$ = 8.38 (s, 2H, $\beta$-H pyrrolic), 8.34 (s, 2H, $\beta$-H pyrrolic), 8.12 (s, 2H, $\beta$-H pyrrolic), 8.01-7.90 (m, 7H, Ar), 7.83-7.76 (m, 3H, Ar), 7.70-7.50 (m, 10H, Ar), 5.77-5.57 (m, 1H, reduced $\beta$-H pyrrolic), 5.39-5.18 (m, 1H, reduced $\beta$-H pyrrolic), 4.17-4.01 (m, 1H, $CH_2$ fused ring), 3.83-3.71 (m, 1H, $CH_2$ fused ring), 3.68-3.55 (m, 1H, $CH_2$ fused ring), 2.62-2.40 (m, 1H, $CH_2$ fused ring) ppm.

In an embodiment the cell culture conditions were as following: The A375 (CRL1619) human melanoma cell lines were purchased from American Type Culture Collection and cultured according to standard procedures using the Dulbecco's Modified Eagle medium (Sigma-Aldrich, Inc; Sigma D-5648) supplemented with 10% heat-inactivated fetal bovine serum (Gibco Invitrogen Life Technologies; Gibco 2010-04), 1% Penicillin-Streptomycin (Gibco Invitrogen Life Technologies; 100 U/mL penicillin and 10 $\mu$g/mL streptomycin - Gibco 15140-122) and 100 $\mu$M sodium pyruvate (Gibco Invitrogen Life Technologies; Gibco 1360) at 37 ºC, in a humidified incubator with 95% air and 5% $CO_2$. For all studies, cells were detached using a solution of 0.25% trypsin-EDTA (Gibco).

In an embodiment the photodynamic activity were as following: For each experiment, A375 cells were plated and kept in the incubator overnight, in order to allow the attachment of the cells. The initial formulation of the sensitizers consisted of a 1 mg/mL solution in DMSO and the desired concentrations were achieved by successive dilutions, being always administered to the cells at 1% DMSO. After sensitizer administration (from 1 nM to 10 $\mu$M), cells were incubated for 24 h. Controls were performed on every test. Cells were washed with PBS and new drug-free medium was added. Each plate was irradiated with a fluence rate of 7.5 mW/cm$^2$ until a total of 10 J was reached, using a light source equipped with a red filter (cut off < 560 nm).

In an embodiment for the evaluation of photocytotoxicity a MTT assay was performed. Cell culture plates were washed and incubated with a solution of 3-(4,5-dimethylthiazol-2-yl)-2,5- diphenyltetrazolium bromide (0.5 mg/mL, Sigma) in PBS, pH 7.4, in the dark at 37 °C for 4 hours. To solubilize formazan crystals, a 0.04 M solution of hydrochloric

acid in isopropanol was added. Absorbance was measured using a Biotek Synergy HT Plate Reader. Cytotoxicity was expressed as the inhibition percentage of cultures subjected to PDT, correlated with cultures only treated with the vehicle administration of sensitizers. This procedure allowed us to establish dose-response curves, obtained using Origin 8.0, and to calculate the concentration of sensitizers that inhibits the proliferation of cultures in 50% ($IC_{50}$). Each experiment was performed in duplicate and repeated in three sets of tests.

In an embodiment the cytotoxicity/viability was carried out: The determination of cellular adenosine triphosphate (ATP) was performed using A375 cells grown in black 96-well plates. Cell viability was assessed with ATPlite one-step assay kit (Perkin Elmer, Inc) according to the manufacturer's instructions using an EnSpire Multimode Plate Reader.

In an embodiment the Luminescence response to $O_2$ Concentration was carried out. In particular, luminescence response to $O_2$ of micellar solution of chlorin **8** were performed using an IVIS Lumina XR optical imaging system equipped with a 150 W Tungsten/Halogen lamp and appropriate filters (Caliper LifeSciences, Hopkinton, MA, USA) excitation/emission pairs as follows: $\lambda$exc = 405 nm $\lambda_{em}$ = 575-650 nm; $\lambda_{em}$ = 695-770 nm. The solutions were saturated with $O_2/N_2$ mixtures with $\%O_2$ = 21, 12, 8, 5.5, 3 and 0.

The term "comprising" whenever used in this document is intended to indicate the presence of stated features, integers, steps, components, but not to preclude the presence or addition of one or more other features, integers, steps, components or groups thereof.

It will be appreciated by those of ordinary skill in the art that unless otherwise indicated herein, the particular sequence of steps described is illustrative only and can be varied without departing from the disclosure. Thus, unless otherwise stated the steps described are so unordered meaning that, when possible, the steps can be performed in any convenient or desirable order.

The disclosure should not be seen in any way restricted to the embodiments described and a person with ordinary skill in the art will foresee many possibilities to modifications thereof.

The above described embodiments are combinable.

The following claims further set out particular embodiments of the disclosure.

**References**

[1] Molecular Imaging in Living Subjects: Seeing Fundamental Biological Processes in a New Light, T. F. Massoud, S.S. Gambhir, Genes and Development, 2003, 17, 545-580.

[2] Review of Long-Wavelength Optical and NIR Imaging Materials: Contrast Agents, Fluorophores, and Multifunctional Nano Carriers, V. Pansare, S. Hejazi, W. J. Faenza, R. K. Prud'homme, Chem. Mater. 2012, 24, 817-827.

[3] Essential Para meters to Consider for the Characterization of Optical Imaging Probes, K. Leung, A. Chopra, L. Shan, W. C. Eckelman, A. E. Menkens, Nanomedicine 2012, 7, 1101-1107.

[4] Fluorescence-enhanced Near Infrared Diagnostic Imaging with Contrast Agents, E. M. Sevick-Muraca, J. P. Houston, M. Gurfinkel, Curr. Opinion Chem. Biol., 2002, 6, 642-650.

[5] In-vivo Near-infrared Fluorescence Imaging, J. V. Frangioni, Curr. Opinion Chem. Biol., 2003, 7, 626-634.

[6] A Review of NIR Dyes in Cancer Targeting and Imaging, S. Luo, E. Zhang, Y. Su, T. Cheng, C. Shi, Biomaterials, 2011, 32, 7127-7138.

[7] Fluorescence in Nanobiotechnology: Sophisticated Fluorophores for Novel Applications, B. Hötzer, I. L. Medintz, N. Hildebrandt, Small, 2012, 8, 2297-2326.

[8] Far-red to Near Infrared Analyte-responsive Fluorescent Probes Based on Organic Fluorophore Platforms for Fluorescence Imaging. L.Yuan, W. Lin, K. Zheng, L. He, W. Huang, Chem.Soc.Rev., 2013, 42, 622-661.

[9] Near-infrared Organic Compounds and Emerging Applications, G. Qian, Z. Y. Wang, Chemistry - An Asian Journal, 2010, 5, 1006-1029.

[10] Convenient synthesis of a highly soluble and stable phosphorescence platinum porphyrin dye. Y. Zems, A. G. Moiseev, D. F. Perepichka, Org. lett., 2013, 15, 5330-5333.

[11] Novel Approach to Chlorins and Bacteriochlorins: [8$\pi$+2$\pi$] Cycloaddition of Diazafulvenium Methides with Porphyrins, N. A. M. Pereira, A. C. Serra, T. M. V. D. Pinho e Melo, Eur. J. Org. Chem., 2010, 6539-6543.

[12] [8$\pi$ +2$\pi$] Cycloaddition of meso-Tetra- and 5,15-Di-Arylporphyrins: Synthesis and Photophysical Characterization of Stable Chlorins and Bacteriochlorins, N. A. M. Pereira, S. M. Fonseca, A. C. Serra, T. M. V. D. Pinho e Melo, H. D. Burrows, Eur. J. Org. Chem. 2011, 3970-3979.

[13] Porphyrins. 18. Luminescence of (Co), (Ni), Pd, Pt Complexes, D. Eastwood, M. Gouterman, J. Mol. Spectroscopy, 1970, 35, 359-375.

[14] Emerging Applications of Phosphorescent Metalloporphyrins, D. B. Papovsky, T. C. O'Riordan, J. Fluorescence, 2005, 15, 569-584.

[15]Photophysical Properties of the New Phosphorescent Platinum(II) and Palladium(II) Complexes of Benzoporphyrins and Chlorins, S. M. Borisov, D. B. Papkovsky, G. V. Ponomarev, A. S. DeToma, R. Saf, I. Klimant, J.Photochem.Photobiol. A: Chemistry, 2009, 206, 87-92

[16] Microwave-promoted insertion of Group 10 metals into free base porphyrins and chlorins: scope and limitations, M. L. Dean, J. R. Schmink, N. E. Leadbeater, C. Brückner, Dalton Trans. 2008, 1341-1345.

[17] A New Generation of Ca2+ Indicators with Greatly Improved Fluoresence Properties, G. Grynkiewicz, M. Poeine, R. Y. Tsien, J. Biol. Chem. 1985, 260, 3440-3450.

[18] Fluorescence Lifetime Imaging, J. R. Lakowicz, H. Szmacinski, K. Nowaczyk, K. W. Berndt, M. Johnson, Anal. Biochem., 1992, 202, 316-330.

[19] A Compilation of Singlet Oxygen Yields from Biologically Relevant Molecules, R. W. Redmond, J. N. Gamlin Photochem. Photobiol., 1999, 70, 391-475.

## Claims

1. Compound of formula I

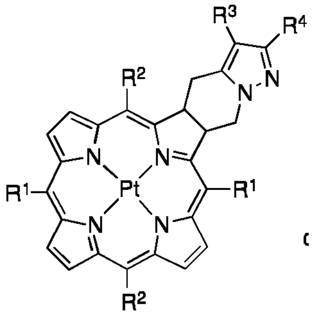

(formula I)

wherein

$R^1$ and $R^2$ are aryl groups,
$R^3$ and $R^4$ are selected from the following list: ester group, or hydroxymethyl group, preferably wherein the ester group is a methyl ester group,
for use in the treatment, therapy or diagnostic of neoplasias or infectious diseases.

2. Compound for use according to the previous claim wherein the aryl group is a phenyl, naphthyl, or combinations thereof, preferably the phenyl group is an unsubstituted phenyl or a substituted phenyl, or preferably the naphthyl group is an unsubstituted naphthyl or a substituted naphthyl.

3. Compound for use according to claim 2 wherein the substituted phenyl group or the substituted naphthyl group is substituted with one or more of the groups consisting of an alkyl, alkoxy, alkylcarboxy, alkylcarbonyl, alkyl ester, cyano, halogen or haloalkyl, preferably wherein the substituted phenyl group is 4-chlorophenyl, 4-methylphenyl, 4-fluorophenyl, 3-methylphenyl or 2,6-dichlorophenyl and $R^3$ and $R^4$ are $CH_2OH$.

4. Compound for use according to any of the previous claims wherein the compound is platinum (II) dimethyl 4,5,6,7-tetrahydropyrazolo[1,5-$\alpha$]pyridine-2,3-dicarboxylate-fused chlorin or platinum (II) 2,3-dihydroxymethyl-4,5,6,7-tetrahydropyrazolo[1,5-$\alpha$]pyridine-fused chlorin.

5. Compound for use according to any of the previous claims wherein the compound is for use in the treatment, therapy or diagnostic of a disease **characterized by** benign or malignant cellular hyperproliferation or by areas of neovascularisation or a cancer, preferably for use in the treatment, therapy or diagnostic of hyperproliferative tissue, in particular hyperproliferative tissue associated to cancer, carcinoma, myeloma, psoriasis, macular degeneration, and/or precancerous conditions or the infectious diseases are caused by microorganisms including virus, bacteria, rickettsia, mycoplasma, protozoa, fungi, or parasites.

6. Compound for use according to any of the previous claims wherein the compound is for use in photodynamic therapy or cytoluminescent therapy.

7. Compound for use according to any of the previous claims wherein the compound is administrated by oral, parenteral, intramuscular, intranasal, sublingual or intratracheal route.

8. Pharmaceutical composition comprising a compound of formula I as described in claims 1-4, preferably further comprising an acceptable carrier, a filler, a binder, a disintegrant or a lubricant wherein acceptable carrier is a transient permeabilizer in the skin.

9. Pharmaceutical composition according to the previous claim 8 comprising a surface penetration enhancer, preferably wherein the surface penetration enhancer is selected from a list consisting of: dialkylsulphoxides, *N*-methylformamide, dimethylformamide, dimethylacetamide, glycols, pyrrolidone derivatives of 1-substituted azacycloalkan-2-ones, or mixtures thereof, more preferably further comprising an anti-viral, an analgesic, an anti-inflammatory agent, a chemotherapy agent, a radiotherapy agent, an antibiotic or a diuretic.

10. Pharmaceutical composition according to any of the previous claims 8-9 for use in intradermal and transdermal therapies.

11. Pharmaceutical composition according to any of the previous claims 8-9 for use in the treatment, therapy or diagnostic of hyperproliferative disorders including benign tumour, cancer, carcinoma, myeloma, macular degeneration, and precancerous conditions, skin disorder selected from actinic keratoses, squamous cell carcinoma, Bowen's disease, psoriasis, acne vulgaris, or rosacea, dermatological condition or acne; preferably wherein cancer is a benign tumour, malignant tumour, early cancer, basal cell carcinoma, cervical dysplasia, soft tissue sarcoma, germ cell tumour, retinoblastoma, age-related macular degeneration, Hodgkin's lymphoma, cancer of the blood, prostate cancer, cervix cancer, uterus cancer, vaginal cancer, breast cancer, naso-pharynx cancer, trachea cancer, larynx cancer, bronchi cancer, bronchioles cancer, lung cancer, hollow organs cancer, esophagus cancer, stomach cancer, bile duct cancer, intestine cancer, colon cancer, colorectum cancer, rectum cancer, bladder cancer, ureter cancer, kidney cancer, liver cancer, gall bladder cancer, spleen cancer, brain cancer, lymphatic system cancer, bone cancer, or skin cancer.

12. Pharmaceutical composition according to any of the previous claims 8-9 for use in the treatment, therapy or diagnostic of a fungal, viral, chlamydial, bacterial, nanobacterial or parasitic infectious disease, preferably for use in the treatment, therapy or diagnostic of HIV, infection with SARS coronavirus, Asian flu virus, herpes simplex, herpes zoster, hepatitis, or viral hepatitis.

13. Kit comprising a compound of formula I as described in claims 1-4 and/or a pharmaceutical composition as described in claims 8-12, preferably further comprising instructions for photodynamic therapy or cytoluminescent therapy, and/or instructions for administration of the composition to a subject.

14. Use of the compound of formula I as described in claims 1-4 as a molecular oxygen sensor or as a biological/biomedical probe.

## Patentansprüche

1. Verbindung der Formel I

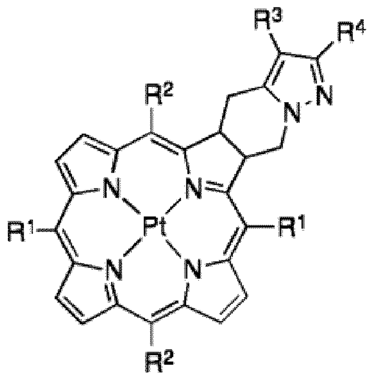

(Formel I)

wobei

$R^1$ und $R^2$ Arylgruppen sind,
$R^3$ und $R^4$ aus folgender Liste ausgewählt werden: Estergruppe oder Hydroxymethylgruppe, wobei die Estergruppe bevorzugt eine Methylestergruppe ist,
zur Verwendung bei der Behandlung, Therapie oder Diagnose von Neoplasien oder Infektionskrankheiten.

**2.** Verbindung zur Verwendung nach dem vorangehenden Anspruch, wobei die Arylgruppe ein Phenyl, Naphthyl oder Kombinationen davon ist, wobei die Phenylgruppe bevorzugt ein unsubstituiertes Phenyl oder ein substituiertes Phenyl ist, oder die Naphthylgruppe bevorzugt ein unsubstituiertes Naphthyl oder ein substituiertes Naphthyl ist.

**3.** Verbindung zur Verwendung nach Anspruch 2, wobei die substituierte Phenylgruppe oder die substituierte Naphthylgruppe mit einer oder mehreren Gruppen substituiert ist, bestehend aus einer Alkyl-, Alkoxy-, Alkylcarboxy-, Alkylcarbonyl-, Alkylester-, Cyano-, Halogen- oder Halogenalkylgruppe, wobei die substituierte Phenylgruppe bevorzugt 4-Chlorphenyl, 4-Methylphenyl, 4-Fluorphenyl, 3-Methylphenyl oder 2,6-Dichlorphenyl ist und $R^3$ und $R^4$ $CH_2OH$ sind.

**4.** Verbindung zur Verwendung nach einem der vorangehenden Ansprüche, wobei die Verbindung Platin(II)-dimethyl 4,5,6,7-tetrahydropyrazolo[1,5-$\alpha$]pyridin-2,3-dicarboxylat-kondensiertes Chlorin oder Platin(II)-2,3-dihydroxymethyl-4,5,6,7-tetrahydropyrazolo[1,5-$\alpha$]pyridin-kondensiertes Chlorin ist.

**5.** Verbindung zur Verwendung nach einem der vorangehenden Ansprüche, wobei die Verbindung zur Verwendung bei der Behandlung, Therapie oder Diagnostik einer Krankheit, die durch gutartige oder bösartige zelluläre Hyperproliferation oder durch Bereiche von Neovaskularisation oder einen Krebs gekennzeichnet ist, bevorzugt zur Verwendung bei der Behandlung, Therapie oder Diagnostik von hyperproliferativem Gewebe, insbesondere von hyperproliferativem Gewebe, das mit einem Krebs, Karzinom, Myelom, Psoriasis, Makuladegeneration und/oder präkanzerösen Zuständen assoziiert ist, oder die Infektionskrankheiten durch Mikroorganismen, einschließlich Viren, Bakterien, Rickettsien, Mykoplasmen, Protozoen, Pilze oder Parasiten verursacht werden.

**6.** Verbindung zur Verwendung nach einem der vorangehenden Ansprüche, wobei die Verbindung zur Verwendung in der photodynamischen Therapie oder der Zytolumineszenz-Therapie bestimmt ist.

**7.** Verbindung zur Verwendung nach einem der vorangehenden Ansprüche, wobei die Verbindung auf oralem, parenteralem, intramuskulärem, intranasalem, sublingualem oder intratrachealem Weg verabreicht wird.

**8.** Pharmazeutische Verbindung, umfassend eine Verbindung der Formel !, wie in den Ansprüchen 1-4 beschrieben, ferner bevorzugt umfassend einen verträglichen Träger, einen Füllstoff, ein Bindemittel, ein Zerfallsmittel oder ein Gleitmittel, wobei der akzeptable Träger ein transienter Permeabilisator in der Haut ist.

**9.** Pharmazeutische Verbindung nach dem vorangehenden Anspruch 8, umfassend einen Oberflächenpenetrationsverstärker, wobei der Oberflächenpenetrationsverstärker bevorzugt aus einer Liste ausgewählt wird, bestehend aus: Dialkylsulfoxiden, N-Methylformamid, Dimethylformamid, Dimethylacetamid, Glykolen, Pyrrolidonderivaten von

1-substituierten Azacycloalkan-2-onen oder Mischungen davon, ferner bevorzugt umfassend ein antivirales Mittel, ein Analgetikum, ein entzündungshemmendes Mittel, ein Chemotherapeutikum, ein Radiotherapeutikum, ein Antibiotikum oder ein Diuretikum.

**10.** Pharmazeutische Verbindung nach einem der vorangehenden Ansprüche 8-9 zur Verwendung in intradermalen und transdermalen Therapien.

**11.** Pharmazeutische Verbindung nach einem der vorangehenden Ansprüche 8-9 zur Verwendung bei der Behandlung, Therapie oder Diagnose von hyperproliferativen Erkrankungen, einschließlich gutartiger Tumore, Krebs, Karzinome, Myelome, Makuladegeneration und präkanzerösen Zuständen, Hauterkrankungen, ausgewählt aus aktinischen Keratosen, Plattenepithelkarzinomen, Morbus Bowen, Psoriasis, Akne vulgaris oder Rosazea, dermatologischen Erkrankungen oder Akne; wobei Krebs bevorzugt ein gutartiger Tumor, ein bösartiger Tumor, ein Frühkarzinom, ein Basalzellkarzinom, eine Gebärmutterhalsdysplasie, ein Weichteilsarkom, ein Keimzellentumor, ein Retinoblastom, eine altersbedingte Makuladegeneration, ein Hodgkin-Lymphom, ein Blutkrebs, ein Prostatakrebs, ein Gebärmutterhalskrebs, ein Gebärmutterkrebs, ein Vaginalkrebs, ein Brustkrebs, ein Nasen-Rachen-Krebs, ein Luftröhrenkrebs, ein Kehlkopfkrebs, Bronchialkrebs, Bronchiolenkrebs, Lungenkrebs, Krebs der Hohlorgane, Speiseröhrenkrebs, Magenkrebs, Gallengangskrebs, Darmkrebs, Dickdarmkrebs, Enddarmkrebs, Blasenkrebs, Harnleiterkrebs, Nierenkrebs, Leberkrebs, Gallenblasenkrebs, Milzkrebs, Hirntumor, Krebs des Lymphsystems, Knochenkrebs oder Hautkrebs ist.

**12.** Pharmazeutische Verbindung nach einem der vorangehenden Ansprüche 8-9 zur Verwendung bei der Behandlung, Therapie oder Diagnose einer Pilz-, Virus-, Chlamydien-, Bakterien-, Nanobakterien- oder parasitären Infektionskrankheit, ferner bevorzugt zur Verwendung bei der Behandlung, Therapie oder Diagnose von HIV, einer Infektion mit dem SARS-Coronavirus, dem asiatischen Grippevirus, Herpes simplex, Herpes zoster, Hepatitis oder Virushepatitis.

**13.** Kit, umfassend eine Verbindung der Formel !, wie in den Ansprüchen 1-4 beschrieben, und/oder eine pharmazeutische Verbindung, wie in den Ansprüchen 8-12 beschrieben, ferner bevorzugt umfassend Anweisungen für die photodynamische Therapie oder die Zytolumineszenztherapie und/oder Anweisungen für die Verabreichung der Verbindung an eine Person.

**14.** Verwendung der Verbindung der Formel !, wie in den Ansprüchen 1-4 beschrieben, als molekularer Sauerstoffsensor oder als biologische/biomedizinische Sonde.

**Revendications**

**1.** Composé de formule I

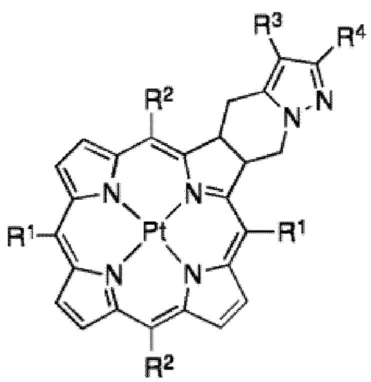

(formule I)

dans laquelle

$R^1$ et $R^2$ sont des groupes aryles,

$R^3$ et $R^4$ sont choisis parmi la liste suivante: groupe ester, ou groupe hydroxyméthyle, préférablement dans laquelle le groupe ester est un groupe ester méthyle,
pour une utilisation dans le traitement, la thérapie ou le diagnostic de néoplasies ou de maladies infectieuses.

**2.** Composé pour une utilisation selon la revendication précédente, dans lequel le groupe aryle est un phényle, un naphtyle, ou des combinaisons de ceux-ci, le groupe phényle étant préférablement un phényle non substitué ou un phényle substitué, ou le groupe naphtyle étant préférablement un naphtyle non substitué ou un naphtyle substitué.

**3.** Composé pour une utilisation selon la revendication 2, dans lequel le groupe phényle substitué ou le groupe naphtyle substitué est substitué par un ou plusieurs groupes consistant en un groupe alkyle, alcoxy, alkylcarboxy, alkylcarbonyle, ester d'alkyle, cyano, halogène ou haloalkyle, préférablement dans lequel le groupe phényle substitué est 4-chlorophényle, 4-méthylphényle, 4-fluorophényle, 3-méthylphényle ou 2,6-dichlorophényle et $R^3$ et $R^4$ sont $CH_2OH$.

**4.** Composé pour une utilisation selon l'une quelconque des revendications précédentes, dans lequel le composé est la chlorine fusionnée à la platine (!!) diméthyle 4,5,6,7-tétrahydropyrazolo[1,5-$\alpha$]pyridine-2,3-dicarboxylate ou la chlorine fusionnée à la platine (!!) 2,3-dihydroxymethyl-4,5,6,7-tetrahydropyrazolo[1,5-$\alpha$]pyridine.

**5.** Composé pour une utilisation selon l'une quelconque des revendications précédentes, dans lequel le composé est pour une utilisation dans le traitement, la thérapie ou le diagnostic d'une maladie **caractérisée par** l'hyperprolifération cellulaire bénigne ou maligne ou par des zones de néovascularisation ou un cancer, préférablement pour une utilisation dans le traitement, la thérapie ou le diagnostic de tissu hyperprolifératif, en particulier de tissu hyperprolifératif associé à un cancer, un carcinome, un myélome, un psoriasis, une dégénérescence maculaire, et/ou à des conditions précancéreuses ou les maladies infectieuses sont causées par des microorganismes incluant des virus, bactéries, rickettsies, mycoplasmes, protozoaires, champignons, ou parasites.

**6.** Composé pour une utilisation selon l'une quelconque des revendications précédentes, dans lequel le composé est pour une utilisation en thérapie photodynamique ou en thérapie cytoluminescente.

**7.** Composé pour une utilisation selon l'une quelconque des revendications précédentes, dans lequel le composé est administré par voie orale, parentérale, intramusculaire, intranasale, sublinguale ou intratrachéale.

**8.** Composition pharmaceutique comprenant un composé de formule I tel que décrit dans les revendications 1-4, préférablement comprenant également un support acceptable, une substance de remplissage, un liant, un désintégrant ou un lubrifiant dans laquelle le support acceptable est un perméabilisant transitoire dans la peau.

**9.** Composition pharmaceutique selon la revendication précédente 8 comprenant un stimulant de pénétration de surface, préférablement dans lequel le stimulant de pénétration de surface est choisi parmi une liste consistant en: dialkylsulphoxides, *N*-méthylformamide, diméthylformamide, diméthylacétamide, glycols, dérivés de pyrrolidone de azacycloalkan-2-ones 1-substitués, ou des mélanges de ceux-ci, plus préférablement comprenant également un antiviral, un analgésique, un agent anti-inflammatoire, un agent de chimiothérapie, un agent de radiothérapie, un antibiotique ou un diurétique.

**10.** Composition pharmaceutique selon l'une quelconque des revendications 8-9 pour une utilisation dans les thérapies intradermiques et transdermiques.

**11.** Composition pharmaceutique selon l'une quelconque des revendications 8-9 pour une utilisation dans le traitement, la thérapie ou le diagnostic de problèmes hyperprolifératifs incluant les tumeurs bénignes, cancers, carcinomes, myélomes, dégénérescences maculaires, et maladies précancéreuses, problèmes de peau choisis parmi les kératoses actiniques, carcinome épidermoïde, maladie de Bowen, psoriasis, acné vulgaris, ou rosacée, problème dermatologique ou acné; préférablement dans laquelle le cancer est une tumeur bénigne, une tumeur maligne, un cancer précoce, un carcinome basocellulaire, une dysplasie cervicale, un sarcome des tissus mous, une tumeur des cellules germinales, un rétinoblastome, une dégénérescence maculaire liée à l'âge, un lymphome de Hodgkin, un cancer du sang, un cancer de la prostate, un cancer du col de l'utérus, un cancer de l'utérus, un cancer vaginal, un cancer du sein, un cancer du nasopharynx, un cancer de la trachée, un cancer du larynx, un cancer des bronches, un cancer des bronchioles, un cancer du poumon, un cancer des organes creux, un cancer de l'œsophage, un cancer de l'estomac, un cancer des canaux biliaires, un cancer de l'intestin, un cancer du côlon, un cancer colorectal, un cancer du rectum, un cancer de la vessie, un cancer de l'uretère, un cancer du rein, un cancer du foie, un cancer

de la vésicule biliaire, un cancer de la rate, un cancer du cerveau, un cancer du système lymphatique, un cancer des os, ou un cancer de la peau.

**12.** Composition pharmaceutique selon l'une quelconque des revendications 8-9 pour une utilisation dans le traitement, la thérapie ou le diagnostic d'une maladie fongique, virale, de chlamydia, bactérienne, nanobactérienne ou infectieuse parasitaire, préférablement pour une utilisation dans le traitement, la thérapie ou le diagnostic du VIH, d'une infection par le SARS coronavirus, du virus de la grippe asiatique, de l'herpès simplex, de l'herpès zoster, de l'hépatite, ou de l'hépatite virale.

**13.** Kit comprenant um composé de formule I tel que décrit dans les revendications 1-4 et/ou une composition pharmaceutique telle que décrite dans les revendications 8-12, préférablement contenant également des instructions pour la thérapie photodynamique ou la thérapie cytoluminescente, et/ou des instructions pour l'administration de la composition à un individu.

**14.** Utilisation du composé de formule I tel que décrit dans les revendications 1-4 en tant que capteur d'oxygène moléculaire ou en tant que sonde biologique/biomédicale.

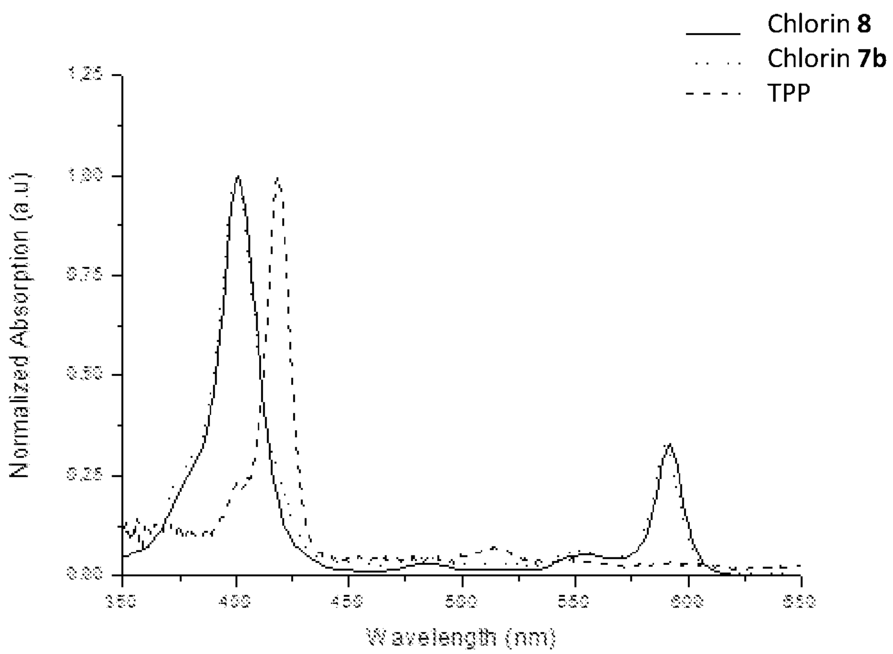

Chlorin **8**
Chlorin **7b**
TPP
Normalized Absorption (a.u)
Wavelength (nm)


Fig.1

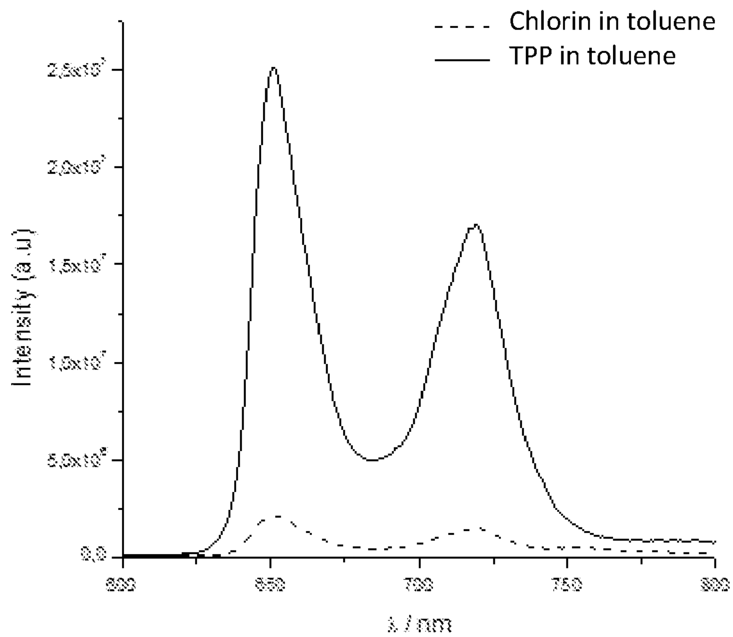

Chlorin in toluene
TPP in toluene
Intensity (a.u)
λ / nm


Fig. 2

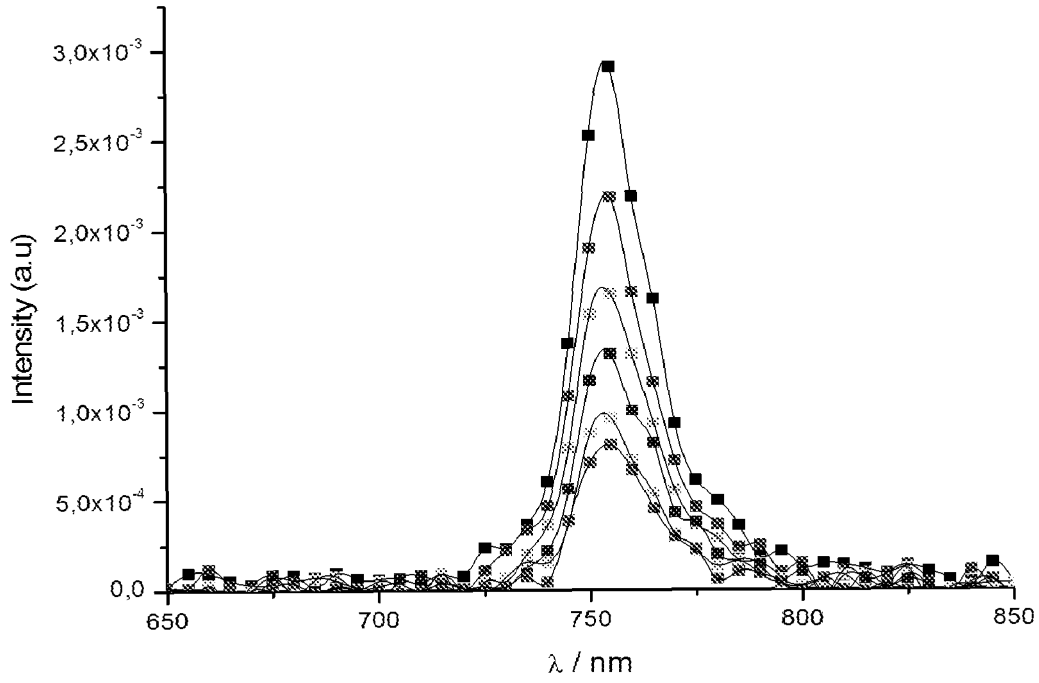
Intensity (a.u)
3,0x10^-3
2,5x10^-3
2,0x10^-3
1,5x10^-3
1,0x10^-3
5,0x10^-4
0,0
650
700
750
800
850
λ / nm

Fig. 3

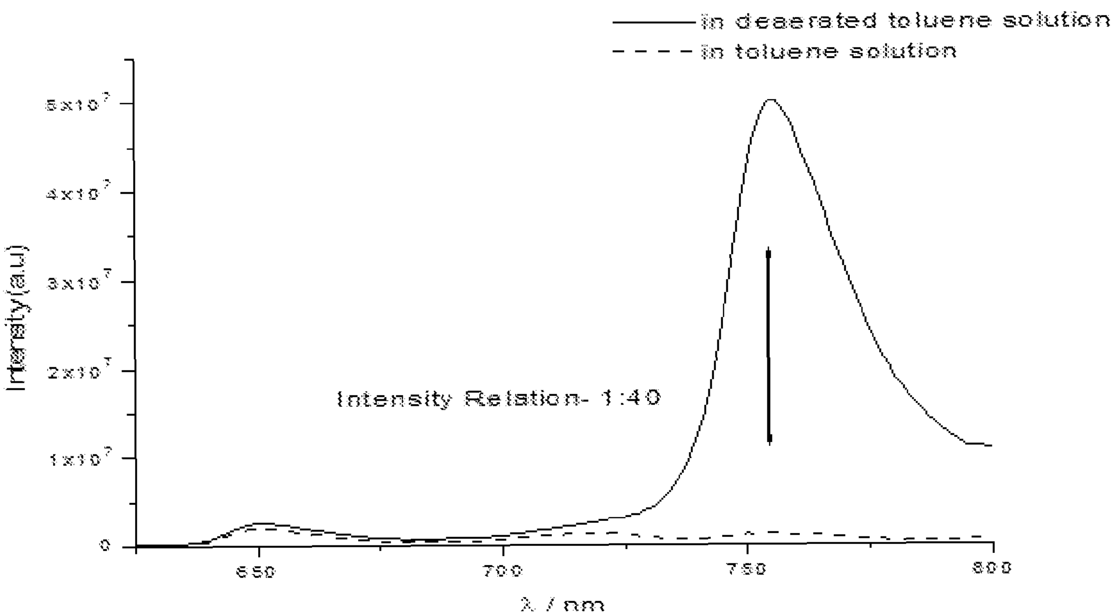
in deaerated toluene solution
in toluene solution
Intensity(a.u)
5x10^7
4x10^7
3x10^7
2x10^7
1x10^7
0
Intensity Relation- 1:40
650
700
750
800
λ / nm

Fig. 4

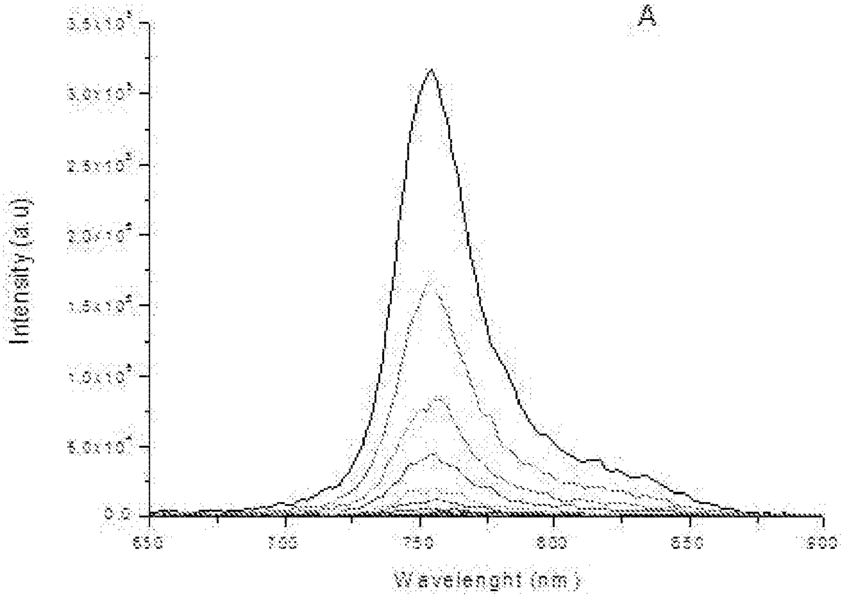
A
Intensity (a.u)
650
700
750
800
850
900
Wavelenght (nm)

Fig. 5A

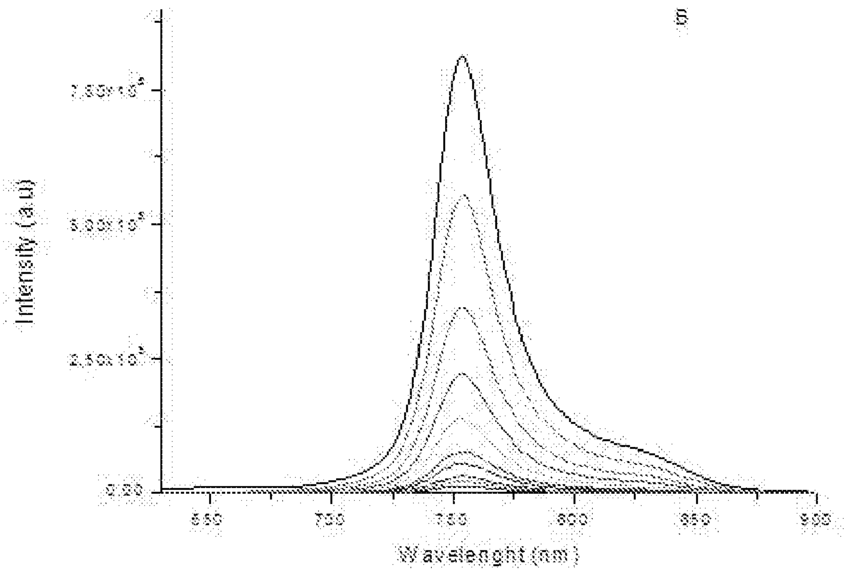
B
Intensity (a.u)
650
700
750
800
850
900
Wavelenght (nm)

Fig. 5B

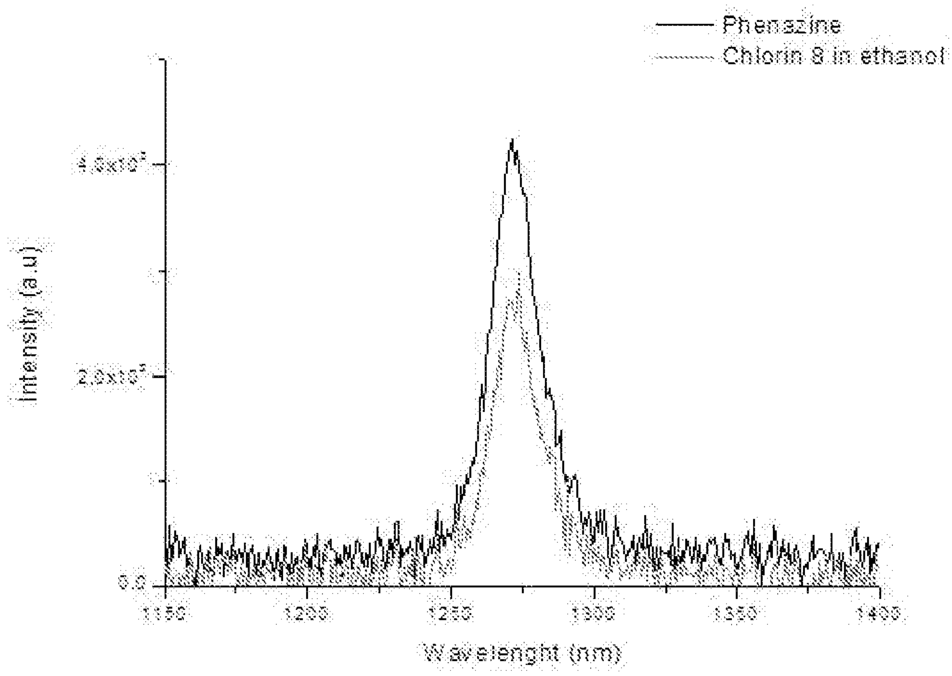
Phenazine
Chlorin 8 in ethanol
4.0x10
2.0x10
0.0
Intensity (a.u)
1150
1200
1250
1300
1350
1400
Wavelenght (nm)

Fig. 6

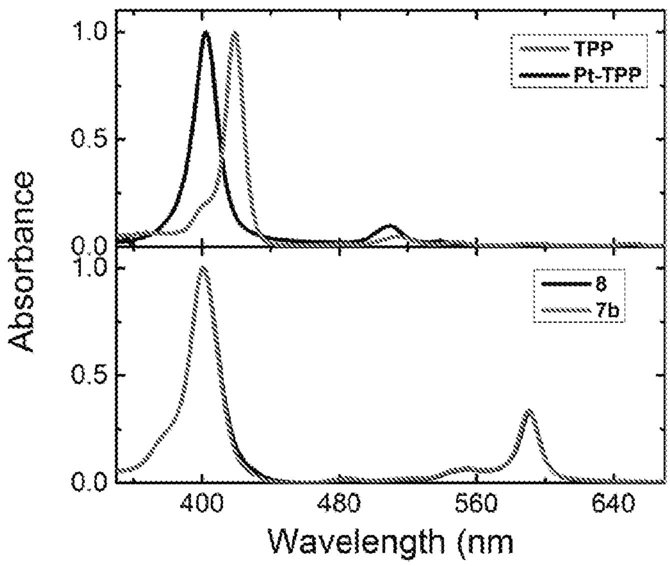
TPP
Pt-TPP
8
7b
1.0
0.5
0.0
Absorbance
400
480
560
640
Wavelength (nm

Fig. 7

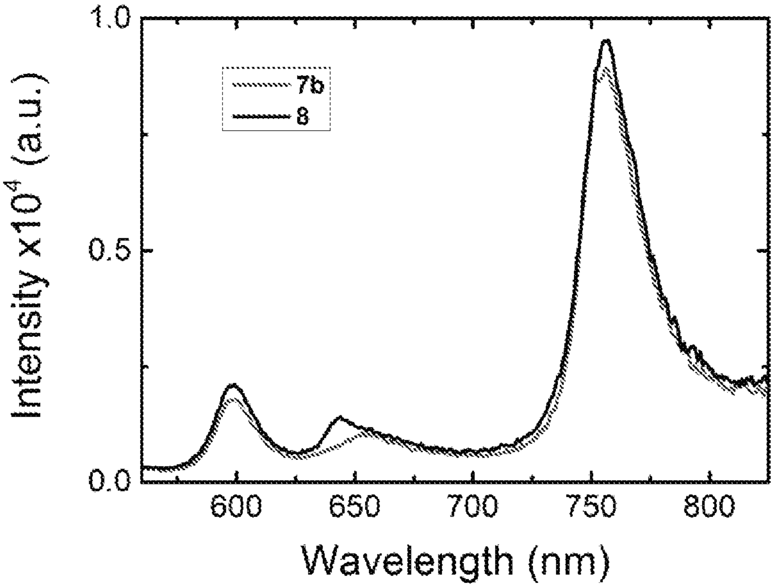
7b
8
Intensity x10^4 (a.u.)
Wavelength (nm)
0.0
0.5
1.0
600
650
700
750
800

Fig. 8

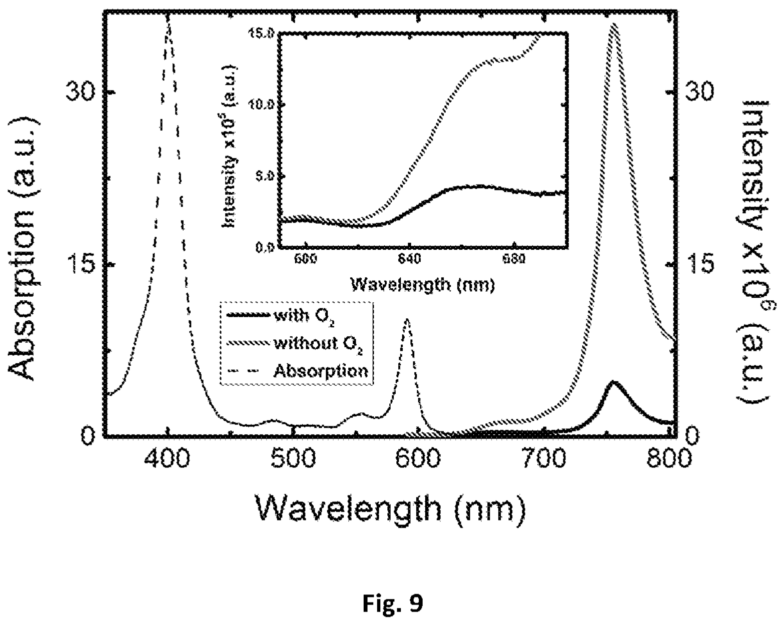
with O2
without O2
Absorption
Absorption (a.u.)
Intensity x10^6 (a.u.)
Wavelength (nm)
0
15
30
400
500
600
700
800

Fig. 9

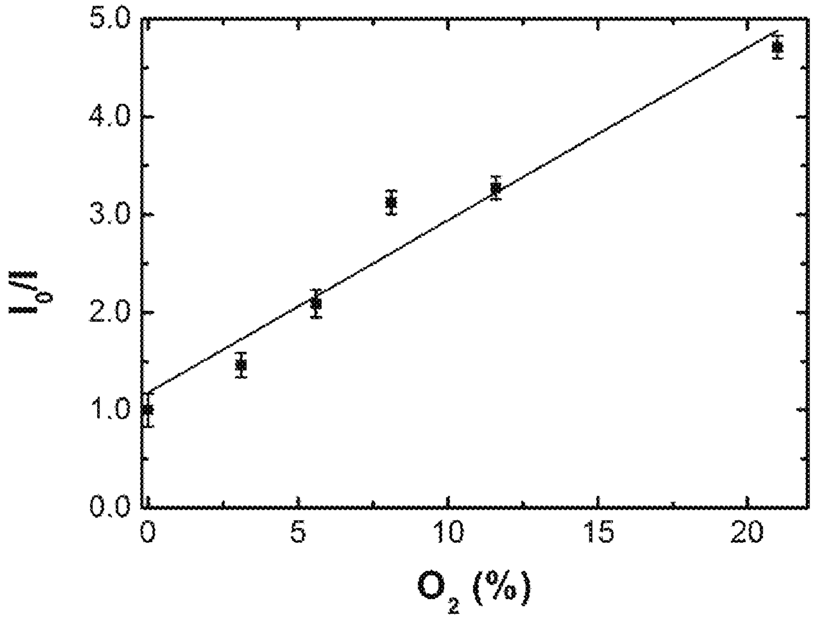
5.0
4.0
3.0
2.0
1.0
0.0
$I_0/I$
0
5
10
15
20
$O_2$ (%)

Fig. 10

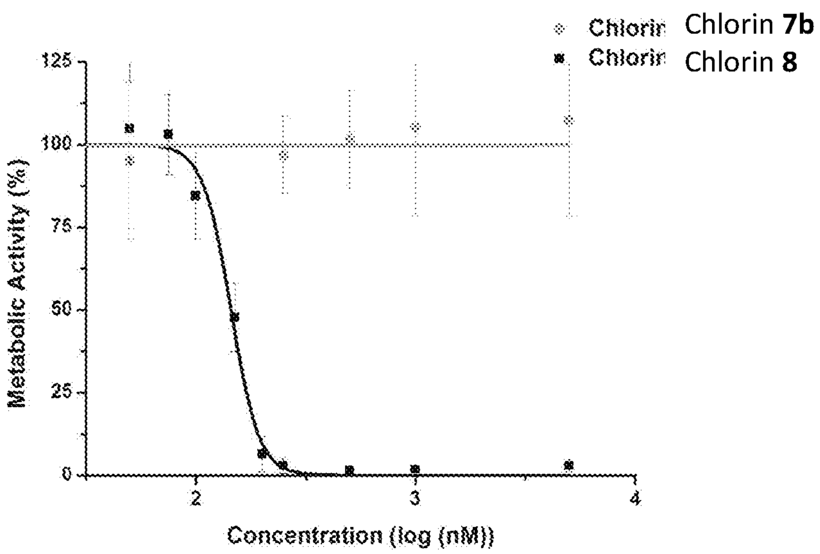
Chlorir Chlorin **7b**
Chlorir Chlorin **8**
125
100
75
50
25
0
Metabolic Activity (%)
2
3
4
Concentration (log (nM))

Fig. 11

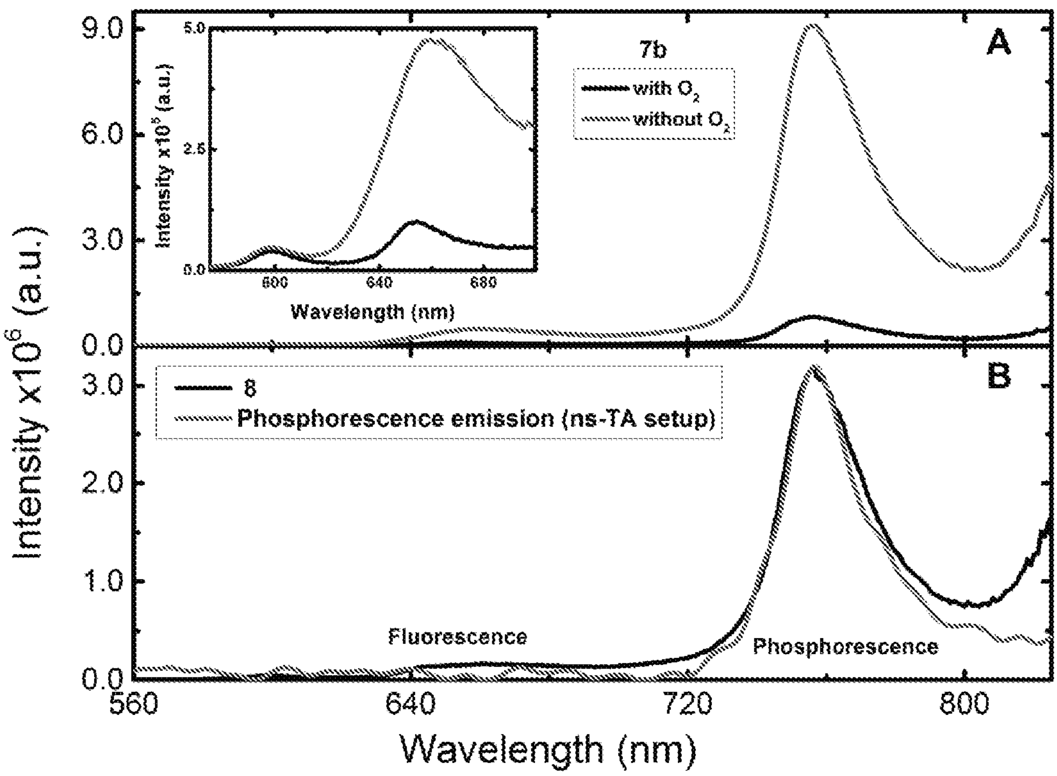
A
7b
with $O_2$
without $O_2$
Intensity x10$^5$ (a.u.)
Wavelength (nm)
B
8
Phosphorescence emission (ns-TA setup)
Fluorescence
Phosphorescence
Intensity x10$^6$ (a.u.)
Wavelength (nm)

Fig. 12

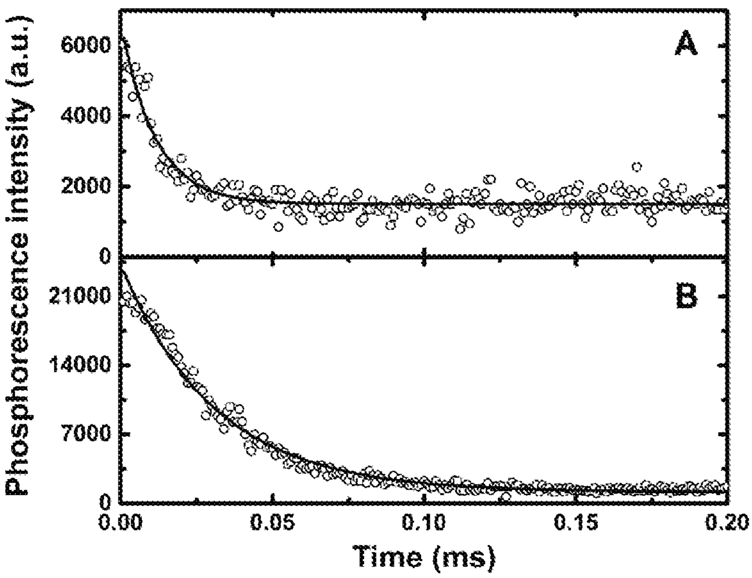
A
B
Phosphorescence intensity (a.u.)
Time (ms)

Fig. 13

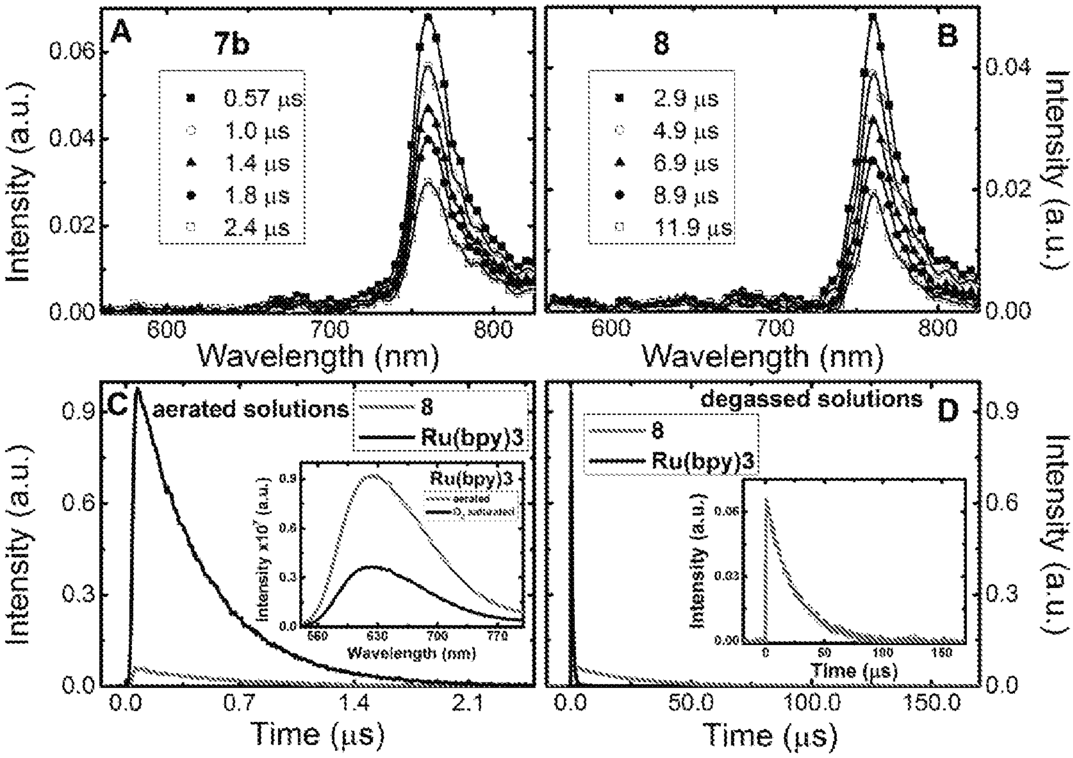
A
7b
0.57 μs
1.0 μs
1.4 μs
1.8 μs
2.4 μs
Intensity (a.u.)
Wavelength (nm)
8
B
2.9 μs
4.9 μs
6.9 μs
8.9 μs
11.9 μs
C
aerated solutions
Ru(bpy)3
Time (μs)
degassed solutions
D


Fig. 14.

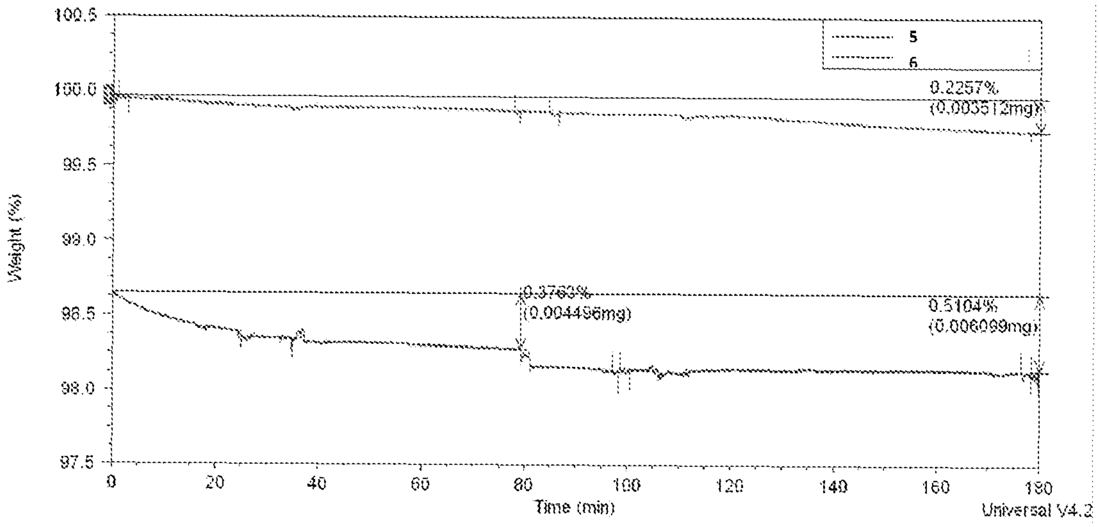
100.5
100.0
99.5
99.0
98.5
98.0
97.5
Weight (%)
5
6
0.2257%
(0.003512mg)
0.3763%
(0.004496mg)
0.5104%
(0.006099mg)
0
20
40
60
80
100
120
140
160
180
Time (min)
Universal V4.2

Fig. 15

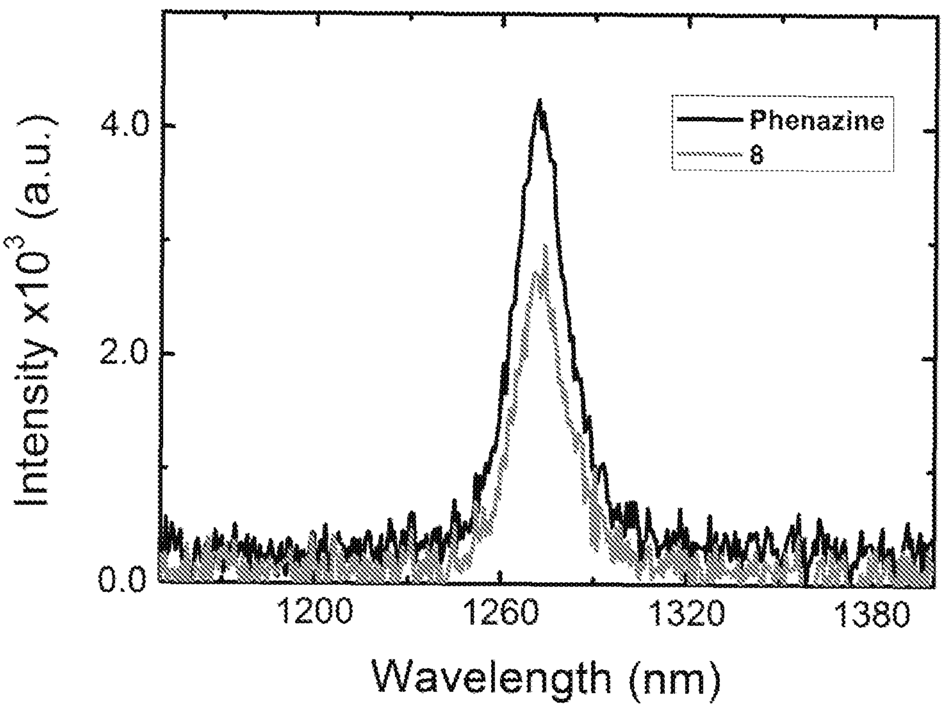
Intensity x10$^3$ (a.u.)
4.0
2.0
0.0
Phenazine
8
1200
1260
1320
1380
Wavelength (nm)

Fig. 16

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **T. F. MASSOUD ; S.S. GAMBHIR.** Molecular Imaging in Living Subjects: Seeing Fundamental Biological Processes in a New Light. *Genes and Development,* 2003, vol. 17, 545-580
- **V. PANSARE ; S. HEJAZI ; W. J. FAENZA ; R. K. PRUD'HOMME.** Review of Long-Wavelength Optical and NIR Imaging Materials: Contrast Agents, Fluorophores, and Multifunctional Nano Carriers. *Chem. Mater,* 2012, vol. 24, 817-827
- **K. LEUNG ; A. CHOPRA ; L. SHAN ; W. C. ECKELMAN ; A. E. MENKENS.** Essential Para meters to Consider for the Characterization of Optical Imaging Probes. *Nanomedicine,* 2012, vol. 7, 1101-1107
- **E. M. SEVICK-MURACA ; J. P. HOUSTON ; M. GURFINKEL.** Fluorescence-enhanced Near Infrared Diagnostic Imaging with Contrast Agents. *Curr. Opinion Chem. Biol.,* 2002, vol. 6, 642-650
- **J. V. FRANGIONI.** In-vivo Near-infrared Fluorescence Imaging. *Curr. Opinion Chem. Biol.,* 2003, vol. 7, 626-634
- **S. LUO ; E. ZHANG ; Y. SU ; T. CHENG ; C. SHI.** A Review of NIR Dyes in Cancer Targeting and Imaging. *Biomaterials,* 2011, vol. 32, 7127-7138
- **B. HÖTZER ; I. L. MEDINTZ ; N. HILDEBRANDT.** Fluorescence in Nanobiotechnology: Sophisticated Fluorophores for Novel Applications. *Small,* 2012, vol. 8, 2297-2326
- **L.YUAN ; W. LIN ; K. ZHENG ; L. HE ; W. HUANG.** Far-red to Near Infrared Analyte-responsive Fluorescent Probes Based on Organic Fluorophore Platforms for Fluorescence Imaging. *Chem.Soc.Rev.,* 2013, vol. 42, 622-661
- **G. QIAN ; Z. Y. WANG.** Near-infrared Organic Compounds and Emerging Applications. *Chemistry - An Asian Journal,* 2010, vol. 5, 1006-1029
- **Y. ZEMS ; A. G. MOISEEV ; D. F. PEREPICHKA.** Convenient synthesis of a highly soluble and stable phosphorescence platinum porphyrin dye. *Org. lett.,* 2013, vol. 15, 5330-5333
- **N. A. M. PEREIRA ; A. C. SERRA ; T. M. V. D. PINHO E MELO.** Novel Approach to Chlorins and Bacteriochlorins: [8π+2π] Cycloaddition of Diazafulvenium Methides with Porphyrins. *Eur. J. Org. Chem.,* 2010, 6539-6543
- **N. A. M. PEREIRA ; S. M. FONSECA ; A. C. SERRA ; T. M. V. D. PINHO E MELO ; H. D. BURROWS.** 8π +2π] Cycloaddition of meso-Tetra- and 5,15-Di-Arylporphyrins: Synthesis and Photophysical Characterization of Stable Chlorins and Bacteriochlorins. *Eur. J. Org. Chem.,* 2011, 3970-3979
- **D. EASTWOOD ; M. GOUTERMAN.** Porphyrins. 18. Luminescence of (Co), (Ni), Pd, Pt Complexes. *J. Mol. Spectroscopy,* 1970, vol. 35, 359-375
- **D. B. PAPOVSKY ; T. C. O'RIORDAN.** Emerging Applications of Phosphorescent Metalloporphyrins. *J. Fluorescence,* 2005, vol. 15, 569-584
- **S. M. BORISOV ; D. B. PAPKOVSKY ; G. V. PONOMAREV ; A. S. DETOMA ; R. SAF ; I. KLIMANT.** Photophysical Properties of the New Phosphorescent Platinum(II) and Palladium(II) Complexes of Benzoporphyrins and Chlorins. *J.Photochem.Photobiol. A: Chemistry,* 2009, vol. 206, 87-92
- **M. L. DEAN ; J. R. SCHMINK ; N. E. LEADBEATER ; C. BRÜCKNER.** Microwave-promoted insertion of Group 10 metals into free base porphyrins and chlorins: scope and limitations. *Dalton Trans.,* 2008, 1341-1345
- **G. GRYNKIEWICZ ; M. POEINE ; R. Y. TSIEN.** A New Generation of Ca2+ Indicators with Greatly Improved Fluoresence Properties. *J. Biol. Chem.,* 1985, vol. 260, 3440-3450
- **J. R. LAKOWICZ ; H. SZMACINSKI ; K. NOWACZYK ; K. W. BERNDT ; M. JOHNSON.** Fluorescence Lifetime Imaging. *Anal. Biochem.,* 1992, vol. 202, 316-330
- **R. W. REDMOND ; J. N. GAMLIN.** A Compilation of Singlet Oxygen Yields from Biologically Relevant Molecules. *Photochem. Photobiol.,* 1999, vol. 70, 391-475